(19) Europäisches Patentamt
European Patent Office
Office européen des brevets

(11) EP 0 780 369 B1

(12) # EUROPÄISCHE PATENTSCHRIFT

(45) Veröffentlichungstag und Bekanntmachung des Hinweises auf die Patenterteilung:
**19.01.2000 Patentblatt 2000/03**

(51) Int Cl.[7]: **C07C 323/32**, A61K 31/135, C07C 215/64, C07C 217/74

(21) Anmeldenummer: **96119283.8**

(22) Anmeldetag: **02.12.1996**

(54) **1-Phenyl-2-dimethylaminomethyl-cyclohexan-1-olverbindungen als pharmazeutische Wirkstoffe**

1-Phenyl-2-dimethylaminomethyl-cyclohexan-1-ol derivatives as pharmaceutical agents

Derivés de 1-phényl-2-diméthylaminaméthyl-cyclohexan-1-ol comme agents pharmaceutiques

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **20.12.1995 DE 19547766**

(43) Veröffentlichungstag der Anmeldung:
**25.06.1997 Patentblatt 1997/26**

(73) Patentinhaber: **Grünenthal GmbH**
**52078 Aachen (DE)**

(72) Erfinder:
- **Graudums, Ivars, Dr.**
  **52222 Stolberg (DE)**
- **Winter, Werner, Prof. Dr.**
  **52078 Aachen (DE)**
- **Frankus, Ernst, Dr.**
  **52223 Stolberg (DE)**
- **Strassburger, Wolfgang Werner Alfred, Prof. Dr.**
  **52146 Würselen (DE)**
- **Friderichs, Elmar Josef, Dr.**
  **52223 Stolberg (DE)**

(56) Entgegenhaltungen:
**BE-A- 616 646** **GB-A- 997 399**

- **CHEMICAL ABSTRACTS, vol. 87, no. 7, 15.August 1977 Columbus, Ohio, US; abstract no. 52866, SAWA, YOICHI ET AL: "1-Substituted 2-(dimethylaminomethyl)-5,5-dimethyl-1-cyc lohexanols" XP002027764 & JP 51 143 650 A (TAKEDA CHEMICAL INDUSTRIES, LTD., JAPAN)**
- **CHEMICAL ABSTRACTS, vol. 101, no. 3, 16.Juli 1984 Columbus, Ohio, US; abstract no. 23053, KATO, TAKESHI ET AL: "Synthesis of N-substituted (6-benzyl-4,4-dimethyl-2-cyclohexenyl)methylamines and related compounds" XP002027765 & CHEM. PHARM. BULL. (1984), 32(1), 44-54 CODEN: CPBTAL;ISSN: 0009-2363, 1984,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft 1-Phenyl-2-dimethylaminomethylcyclohexan-1-olverbindungen, Verfahren zu deren Herstellung sowie die Verwendung dieser Verbindungen in Arzneimitteln.

Die Behandlung chronischer und nicht chronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für die Patienten zu verstehen ist, dokumentiert sich in der großen Zahl von wissenschaftlichen Arbeiten, die auf dem Gebiet der Analgetik sowie der Grundlagenforschung zur Nociception in letzter Zeit erschienen sind.

Opioide werden seit vielen Jahren zur Schmerzbehandlung eingesetzt, obwohl sie eine Reihe von Nebenwirkungen, beispielsweise Abhängigkeit, Atemdepression, gastrointestinale Hemmwirkung und Obstipation, hervorrufen. Es sind daher bestimmte Vorsichtsmaßnahmen, beispielsweise spezielle Verordnungsvorschriften, erforderlich, um Opioide über einen längeren Zeitraum oder in höheren Dosierungen therapeutisch einsetzen zu können (Goodman, Gilman The Pharmacological Basis of Therapeutics" Pergamon Press, New York. 1990).

Tramadolhydrochlorid - (1RS, 2RS)-2-Dimethylaminomethyl-1-(3-methoxyphenyl)-cyclohexanol, Hydrochlorid - nimmt unter den zentral wirksamen Analgetika eine Sonderstellung ein, da dieser Wirkstoff eine starke Schmerzhemmung ohne die für Opioide bekannten Nebenwirkungen hervorruft (J. Pharmacol. Exp. Ther. 267, 331 (1993)). Tramadol ist ein Racemat und besteht aus gleichen Mengen an (+)- und (-)-Enantiomer. In vivo bildet der Wirkstoff den Metaboliten O-Desmethyl-tramadol, der ebenfalls als Enantiomerengemisch vorliegt. Untersuchungen haben ergeben, daß sowohl die Enantiomeren von Tramadol als auch die Enantiomeren der Tramadolmetaboliten an der analgetischen Wirkung beteiligt sind (J. Pharmacol. Exp. Ther. 260, 275 (1992)).

Aus An. Quim., 69(7-8), 915-920 (1973) sind 1,2,4,4-tetrasubstituierte Cyclohexanolverbindungen bekannt, die als Spasmolytika Verwendung finden.

Die belgische Patentschrift BE 616,646 beschreibt 1,2,4,4-tetrasubstituierte Cyclohexanolverbindungen mit antitussiver Wirkung.

In Arzneimittel-Forsch. 13, 991-999 (1963) werden 1,2,4-trisubstituierte Cyclohexanolverbindungen offenbart. Einige dieser Verbindungen haben eine spasmolytische Wirkung.

Die der Erfindung zugrundeliegende Aufgabe bestand in der Entwicklung von analgetisch wirksamen Substanzen, die sich zur Behandlung starker Schmerzen eignen, ohne die für Opioide typischen Nebenwirkungen hervorzurufen. Darüber hinaus sollten die zu entwickelnden Substanzen nicht die während der Behandlung mit Tramadol in manchen Fällen auftretenden Nebenwirkungen, beispielsweise Übelkeit und Erbrechen, besitzen.

Es wurde nun gefunden, daß von bestimmten 1-Phenyl-2-dimethylaminomethyl-cyclohexan-1-olverbindungen die an die zu entwickelnden Substanzen gestellten Anforderungen erfüllt werden. Diese Verbindungen zeichnen sich durch eine im Vergleich zu Tramadol stark erhöhte analgetische Wirkung aus. Sie sind somit auch bei besonders starken Schmerzzuständen einsetzbar, bei denen eine mittelstarke Opioidwirkung nicht mehr ausreichend ist. Die Verbindungen können daher in geringeren Dosen appliziert werden, wodurch eine Reduzierung der unspezifischen Nebenwirkungen ermöglicht wird. Darüber hinaus lassen sich durch die stärkere analgetische Wirkung auch andere Anwendungsgebiete im Bereich der Analgetik erschließen, die von der mittelstarken Opioidwirkung des Tramadols nicht abgedeckt werden, beispielsweise die balancierte Narkose oder starke oder sehr starke Schmerzzustände im perioperativen Bereich.

Gegenstand der Erfindung sind dementsprechend 1-Phenyl-2-dimethylaminomethyl-cyclohexan-1-olverbindungen der Formel I

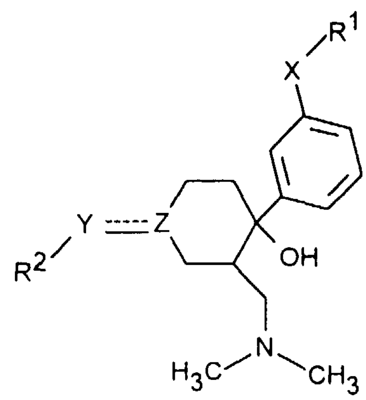

in der X O oder S bedeutet,
$R^1$ H, $C_{1-6}$-Alkyl, $C_{2-6}$-Alkenyl, $C_{5-7}$-Cycloalkyl oder halogeniertes $C_{1-6}$-Alkyl bedeutet,
die Gruppierung

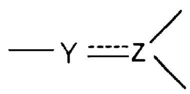

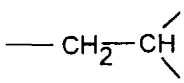 , 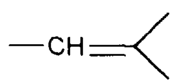 oder 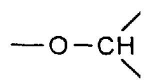

bedeutet,
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,
in Form ihrer Basen oder Salze von physiologisch verträglichen Säuren.

Bevorzugte 1-Phenyl-2-dimethylaminomethyl-cyclohexan-1-ole stellen Verbindungen der Formel I dar, in denen
$R^1$ H, $C_{1-4}$-Alkyl, 2'-Methyl-2'-propenyl, Cyclopentyl oder Fluorethyl bedeutet mit der Maßgabe, daß $R^1$ $C_{1-4}$-Alkyl ist, wenn X S bedeutet, und
$R^2$ $C_{1-4}$-Alkyl, $C_{2-4}$-Alkenyl, Cyclopentylmethyl, Phenyl, $C_{1-4}$-Alkoxyphenyl, Benzyl, $C_{1-4}$-Alkylbenzyl, einfach oder doppelt halogeniertes Phenyl oder einfach oder doppelt halogeniertes Benzyl bedeutet.

Verbindungen der Formel I, in denen
$R^1$ H, Methyl, Ethyl, Isopropyl, 2'-Methyl-2'-propenyl, Cyclopentyl oder Fluorethyl bedeutet, mit der Maßgabe, daß $R^1$ Methyl ist, wenn X S bedeutet, und
$R^2$ Methyl, Propyl, 2'-Methyl-propyl, Allyl, 2'-Methyl-2'-propenyl, Cyclopentylmethyl, Phenyl, 3-Methoxyphenyl, Benzyl, 4-tert-Butylbenzyl, 4-Chlorbenzyl, 4-Fluorbenzyl oder 3,4-Dichlorbenzyl bedeutet, sind besonders bevorzugte 1-Phenyl-2-dimethylaminomethyl-cyclohexan-1-olverbindungen.

Insbesonders bevorzugt werden 1-Phenyl-2-dimethylaminomethyl-cyclohexan-1-ole der Formel I, in denen
$R^1$ H, Methyl oder Cyclopentyl bedeutet, mit der Maßgabe, daß $R^1$ Methyl ist, wenn X S bedeutet,
die Gruppierung

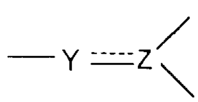

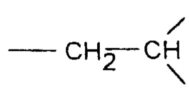 oder 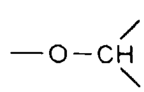

bedeutet
und
$R^2$ Cyclopentylmethyl, Benzyl und 4-Chlorobenzyl darstellt.

Ausgewählte 1-Phenyl-2-dimethylaminomethyl-cyclohexan-1-olverbindungen sind Verbindungen der Formel I, in denen X O ist, $R^1$ H oder Methyl bedeutet,
die Gruppierung

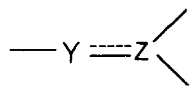

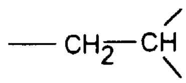

oder

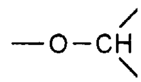

bedeutet und
$R^2$ Benzyl ist.

1-Phenyl-2-dimethylaminomethyl-cyclohexan-1-olverbindungen in Form ihrer Diastereomeren haben vorzugsweise die Konfiguration der Formel Ia

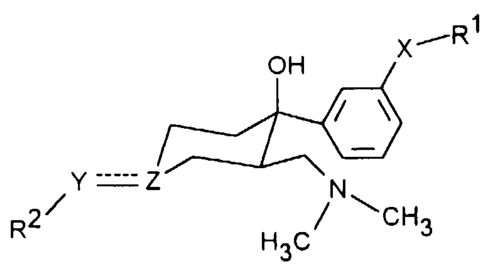

in der der Phenylring und die Dimethylaminomethyl-Gruppe trans zueinander stehen.

Weiterer Erfindungsgegenstand ist ein Verfahren zur Herstellung einer 1-Phenyl-2-dimethylaminomethyl-cyclohexan-1-olverbindung der Formel I, in der
X O oder S bedeutet,
$R^1$ $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet,
die Gruppierung

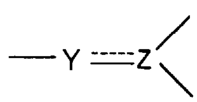

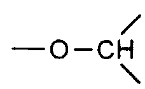

ist,
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,
welches dadurch gekennzeichnet ist, daß man ein 4-substituiertes Cyclohexan-1-on der Formel IIa

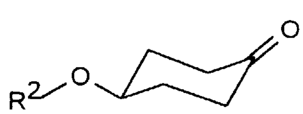

herstellt, indem man
entweder eine Verbindung der Formel IV

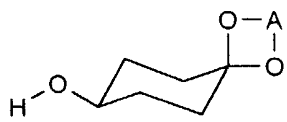

in der A ein verzweigter oder unverzweigter $C_nH_{2n}$-Rest ist und n eine ganze Zahl zwischen 2 und 6 bedeutet, mit einer Verbindung der Formel III

$$R^2—G$$

in der G Cl, Br, I oder Toluolsulfonyloxy ist, alkyliert und anschließend mittels Protonenkatalyse deacetalisiert, oder eine Verbindung der Formel V

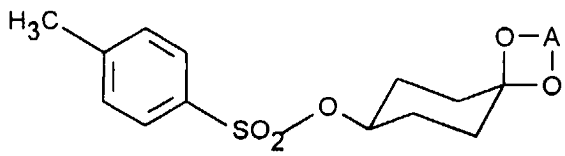

mit einem Alkoholat, das man aus einem Alkohol der Formel VI

$$R^2—OH$$

herstellt, alkyliert und anschließend mittels Protonenkatalyse zu einer Verbindung der Formel IIa deacetalisiert, die erhaltene Verbindung der Formel IIa entweder mit Dimethylamin in einer Mannich-Reaktion oder mit Dimethylammoniummethylenchlorid zu einer Verbindung der Formel VIIa

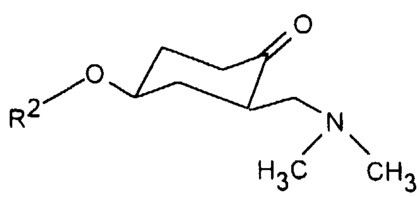

umsetzt, die man mit einer metallorganischen Verbindung der Formel VIII

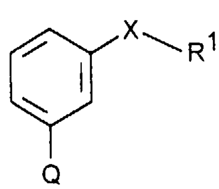

in der Q MgCl, MgBr, MgI oder Li bedeutet, in eine Verbindung der Formel I überführt.

Die Umsetzung einer Verbindung der Formel IV mit einer Verbindung der Formel III wird durchgeführt, in dem man in ein inertes, polares Lösungsmittel, z.B. Dimethylformamid, ein Hydrid wie NaH oder ein Alkoholat wie Kalium-tert-butylat und anschließend eine Verbindung der Formel IV gibt und bei Temperaturen zwischen 20 °C und 60° C

rührt. Dann wird eine Verbindung der Formel III zugegeben und bei Temperaturen zwischen 20° C und 120° C alkyliert. Zur Herstellung des Ketons IIa wird die alkylierte Verbindung IV in einem Ether, beispielsweise Diethylether, Diisopropylether, Tetrahydrofuran, Dioxan, oder in einem Kohlenwasserstoff gelöst und unter Rühren mit Säuren wie HCl, HBr, $H_2SO_4$ deacetalisiert (Gray et al., J. Org. Chem. 35 (1970), 1525; Krepcho et al., J. Org. Chem. 36 (1971), 146).

Eine weitere Möglichkeit ein Keton der Formel IIa herzustellen, besteht in der Alkylierung eines von einem Alkohol der Formel VI abgeleiteten Alkoholats in einem polaren, inerten Lösungsmittel, beispielsweise Dimethylformamid, mit einer Verbindung der Formel V und anschließender Deacetalisierung. Die Deacetalisierung wird ebenfalls unter den vorstehend angegebenen Bedingungen durchgeführt. Das Alkoholat eines Alkohols der Formel VI erhält man durch Umsetzung mit beispielsweise NaH, einem Alkoholat, NaOH oder KOH.

Mit einer Verbindung der Formel IIa läßt sich bei Temperaturen zwischen 40° C und 120° C mit Dimethylamin eine Mannich-Reaktion durchführen, wobei eine Verbindung der Formel VIIa entsteht. Als Lösungsmittel dienen geradkettige oder verzweigte $C_{1-4}$-Alkohole oder Essigsäure. Formaldehyd kann als Formalinlösung oder als Paraformaldehyd eingesetzt werden (J. R. Hwu et al., J. Chem. Soc., Chem. Commun. 1984, 721).

Ein Keton der Formel VIIa in Form des Hydrochlorids kann auch erhalten werden, in dem man ein Keton der Formel IIa mit Dimethylammoniummethylenchlorid in aprotischen Lösungsmitteln, beispielsweise Acetonitril, bei Temperaturen zwischen 20° C und 40° C umsetzt.

Die Reaktion eines Ketons der Formel VIIa mit einer Grignard-Verbindung der Formel VIII oder mit einer lithiumorganischen Verbindung der Formel VIII kann in aliphatischen Ethern, beispielsweise Diethylether und/oder Tetrahydrofuran, bei einer Temperatur zwischen 30° C und 80° C durchgeführt werden. Lithiumorganische Verbindungen der Formel VIII, die für die vorstehende Reaktion einsetzbar sind, lassen sich durch Umsetzung einer Verbindung der Formel VIII, in der Q Cl, Br oder I bedeutet, mit beispielsweise einer n-Butyllithium/Hexan-Lösung durch Halogen/Lithiumaustausch erhalten.

Ferner ist Erfindungsgegenstand ein Verfahren zur Herstellung einer 1-Phenyl-2-dimethylaminomethyl-cyclohexan-1-olverbindung der Formel I in der
X O oder S bedeutet,
$R^1$ $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet,
die Gruppierung

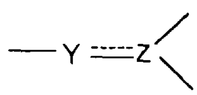

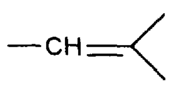

ist
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,
welches dadurch gekennzeichnet ist, daß man ein 4-substituiertes Cyclohexan-1-on der Formel IIb

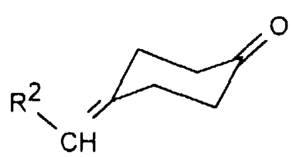

herstellt, indem man entweder eine Grignard-Verbindung der Formel IX

$$R^2\text{-}CH_2\text{-Mg-Hal}$$

in der Hal Cl, Br, oder I bedeutet, mit einem Keton der Formel X

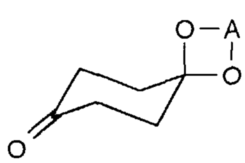

in der A ein verzweigter oder unverzweigter $C_nH_{2n}$-Rest mit n eine ganze Zahl zwischen 2 und 6 ist, zu einer Verbindung der Formel XI

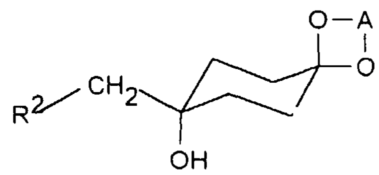

umsetzt, aus dieser anschließend mittels protonenkatalysierter Deacetalisierung eine Keto-Verbindung der Formel XII

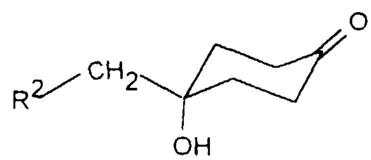

herstellt und durch anschließende Dehydratisierung die Verbindung der Formel IIb erhält oder ein Keton der Formel X mit einem Phosphoran der Formel XIII

$$R_3P{=}CH{-}R^2$$

in dem R Aryl bedeutet, nach Wittig zu einer Verbindung der Formel XIV

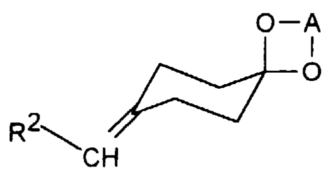

umsetzt, die man anschließend mittels Protonenkatalyse in ein Keton der Formel IIb überführt, die erhaltene Verbindung der Formel IIb danach entweder mit Dimethylamin in einer Mannich-Reaktion oder mit Dimethylammoniummethylenchlorid zu einer Verbindung der Formel VIIb

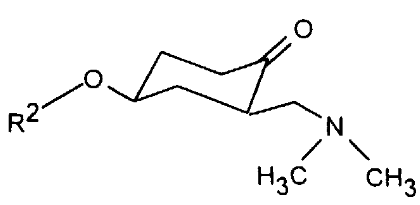

umsetzt, die man mit einer metallorganischen Verbindung der Formel VIII, in der Q MgCl, MgBr, MgI oder Li bedeutet, in eine Verbindung der Formel I überführt.

Weiterer Erfindungsgegenstand ist ein Verfahren zur Herstellung einer 1-Phenyl-2-dimethylaminomethyl-cyclohexan-1-olverbindung der Formel I in der

X O oder S bedeutet, $R^1$ $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet,

die Gruppierung

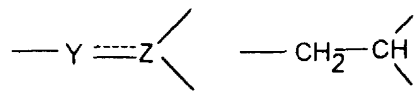

ist,
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,
welches dadurch gekennzeichnet ist, daß man eine analog dem vorstehend beschriebenen Verfahren hergestellte Verbindung der Formel IIb in Gegenwart eines Pd- oder Pt-Katalysators zu einem 4-substituierten Cyclohexan-1-on der Formel IIc

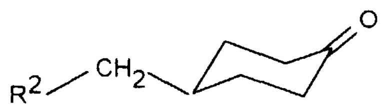

hydriert, die erhaltene Verbindung der Formel IIc entweder mit Dimethylamin in einer Mannich-Reaktion oder mit Dimethylammoniummethylenchlorid zu einer Verbindung der Formel VIIc

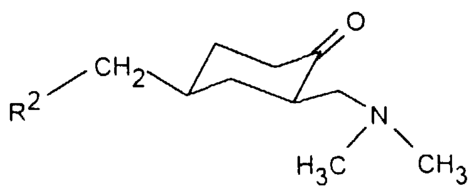

umsetzt, die man mit einer metallorganischen Verbindung der Formel VIII, in der Q MgCl, MgBr, MgI oder Li bedeutet, in eine Verbindung der Formel I überführt.

Eine Verbindung der Formel XI läßt sich durch Umsetzung einer Verbindung der Formel IX mit einer Verbindung der Formel X in aliphatischen oder cyclischen Ethern, beispielsweise Diethylether oder Tetrahydrofuran bei Temperaturen zwischen 30° C und 100° C herstellen. Zur Erhöhung der Ausbeute der Verbindung XI kann beispielsweise 1,2-Dibromethan zugegeben werden. Durch Abspaltung der Acetalgruppe aus der Verbindung der Formel XI erhält man dann die entsprechende Verbindung der Formel XII. Mit Ameisensäure, Acetanhydrid oder einem anorganischen Säurechlorid wird die Verbindung der Formel XII bei Temperaturen zwischen 20° C und 120° C zu einem Olefingemisch umgesetzt. Aus dem Olefingemisch läßt sich die Verbindung IIb durch bekannte Trennverfahren, beispielsweise mittels Säulenchromatographie, isolieren.

Gewünschtenfalls kann man das gesamte Olefingemisch in Gegenwart eines Pt- oder Pd-Katalysators in Essigsäure oder einem geradkettigen oder verzweigten $C_{1-4}$-Alkohol bei 1-100 atm und Temperaturen zwischen 20° C und 100° C zu einer Verbindung der Formel IIc hydrieren (Shiotani et. al., Chem. Pharm. Bull., 20 (1972), 277).

Eine weitere Möglichkeit eine Verbindung der Formel IIb oder IIc zu erhalten, besteht in der Wittig-Umsetzung eines Ketons der Formel X mit einem Phosphoran der Formel XIII, in der R Aryl, beispielsweise Phenyl ist, zu einer Verbindung mit der Formel XIV. Die Reaktion führt man üblicherweise in einem cyclischen Ether, beispielsweise Tetrahydrofuran, oder einem Kohlenwasserstoff, beispielsweise Toluol, bei Temperaturen zwischen 50° C und 110° C durch. Die erhaltene Verbindung der Formel XIV wird wie vorstehend beschrieben deacetalisiert und dehydratisiert. Die erhaltene Verbindung der Formel IIb kann gewünschtenfalls zu einer Verbindung der Formel IIc hydriert werden.

Weiterer Gegenstand der Erfindung ist ein Verfahren zur Herstellung einer 1-Phenyl-2-dimethylaminomethyl-cyclohexan-1-olverbindung der Formel I in der X O oder S bedeutet, $R^1$ H darstellt,
die Gruppierung

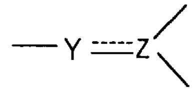

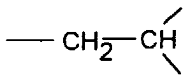 , 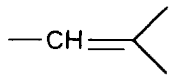 oder 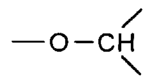

bedeutet,
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,
wobei das Verfahren dadurch gekennzeichnet ist, daß man entweder eine Verbindung der Formel I, in der $R^1$ Methyl bedeutet, mit Diisobutylaluminiumhydrid umsetzt oder eine Verbindung der Formel I, in der $R^1$ Benzyl ist, in Gegenwart eines Pt- oder Pd-Katalysators hydriert oder eine Verbindung der Formel I, in der $R^1$ Diarylalkylsilyl oder Trialkylsilyl bedeutet, hydrolysiert oder mit Tetra-n-butylammoniumfluorid umsetzt.

Die Reaktion einer Verbindung der Formel I, in der $R^1$ Methyl und X O ist, mit Diisobutylaluminiumhydrid wird vorzugsweise in aromatischen Lösungsmitteln, beispielsweise Toluol, bei Temperaturen zwischen 60° C und 130° C durchgeführt.

Die Hydrierung einer Verbindung der Formel I, in der $R^1$ Benzyl und X O ist, wird üblicherweise in Gegenwart eines Pt- oder Pd-Katalysators in Essigsäure oder einem verzweigten oder unverzweigten $C_{1-4}$-Alkohol bei 1 - 100 atm und Temperaturen zwischen 20° C und 50° C durchgeführt.

Liegt als Ausgangsverbindung eine Verbindung der Formel I vor, bei der $R^1$ ein Diarylalkylsilyl- oder Trialkylsilylrest, vorzugsweise tert-Butyldimethylsilyl oder tert-Butyldiphenylsilyl ist, wird mit Säuren, beispielsweise verdünnter Salzsäure oder mit Tetra-n-butylammoniumfluorid die Silyl-Gruppe abgespalten.

Ferner ist Erfindungsgegenstand ein Verfahren zur Herstellung einer 1-Phenyl-2-dimethylaminomethyl-cyclohexan-1-olverbindung der Formel I in der
X O oder S bedeutet,
$R^1$ H, $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet,
die Gruppierung

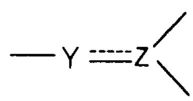

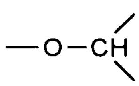 , 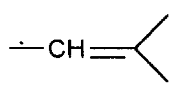 oder 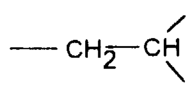

bedeutet,
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,
welches dadurch gekennzeichnet ist, daß man ein Keton der Formel XV

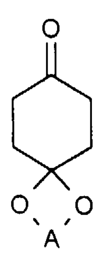

in der A ein verzweigter oder unverzweigter $C_nH_{2n}$-Rest mit n eine ganze Zahl zwischen 2 und 6 ist,
mit Dimethylammoniummethylenchlorid zu einem β-Dimethylaminoketon der Formel XVI

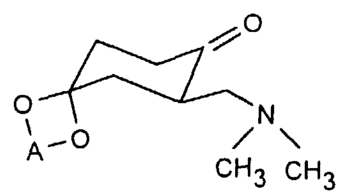

umsetzt, aus dem man anschließend mit einer metallorganischen Verbindung der Formel VIII, in der Q MgCl, MgBr, MgI oder Li bedeutet, eine Verbindung der Formel XVII

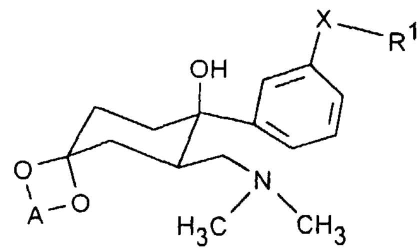

herstellt, die man anschließend mittels Protonenkatalyse zu einer Verbindung der Formel XVIII

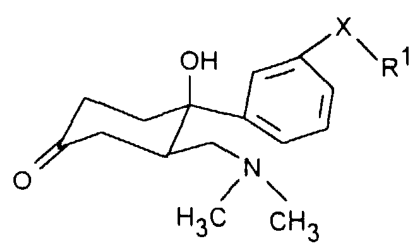

deacetalisiert, entweder die erhaltene Verbindung der Formel XVIII zu einem 4-Hydroxy-Derivat der Formel XIX

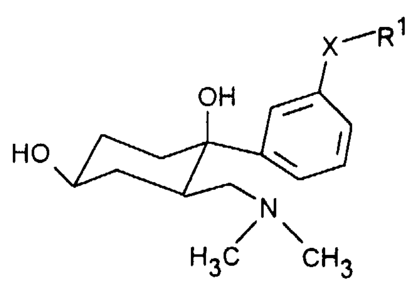

reduziert, aus dem man durch anschließende Alkoholatbildung und Umsetzung mit einer Verbindung der Formel III, in der G Cl, Br, I oder Toluolsulfonyloxy ist, eine Verbindung der Formel I herstellt, in der die Gruppierung

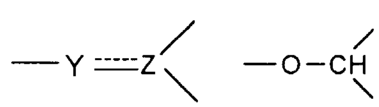

ist,
oder die erhaltene Verbindung der Formel XVIII mit einer Verbindung der Formel XX

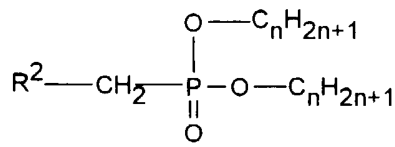

in der n eine ganze Zahl von 1 - 3 bedeutet, zu einer Verbindung der Formel I, in der
die Gruppierung

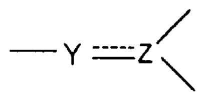 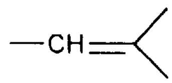

ist,
umsetzt, die man gewünschtenfalls zu einer Verbindung der Formel I, in der
die Gruppierung

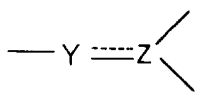 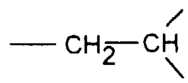

ist,
hydriert.

Die Umsetzung der Keto-Verbindung XV mit Dimethylmethylenimmoniumchlorid zu einem β-Dimethylaminoketon mit spirocyclischer Acetalstruktur der Formel XVI wird üblicherweise in Acetonitril unter Acetylchloridkatalyse durchgeführt. Anschließend wird die erhaltene Verbindung der Formel XVI mit einer metallorganischen Verbindung der Formel VIII in aliphatischen oder cyclischen Ethern, beispielsweise Diethylether oder Tetrahydrofuran bei Temperaturen zwischen 30° C und 80° C zu einer Verbindung der Formel XVII umgesetzt. Zur Erhöhung der Ausbeute von Verbindung XVII kann beispielsweise 1,2-Dibromethan zugegeben werden.

Die erhaltene Verbindung der Formel XVII löst man in einem Ether, z.B. Diethylether, Diisopropylether, Tetrahydrofuran, Dioxan, oder einem Kohlenwasserstoff. Anschließend wird mit Säuren, beispielsweise HCl, HBr oder $H_2SO_4$, die spirocyclische Acetal-Gruppe unter Rühren abgespalten und eine Verbindung der Formel XVIII erhalten. Die anschließende Reduktion der Verbindung der Formel XVIII zu einem 4-Hydroxyderivat der Formel XIX läßt sich mit einem komplexen Alkalimetallhydrid, vorzugsweise Natriumborhydrid oder Lithiumaluminiumhydrid, in einem organischen Lösungsmittel, beispielsweise Tetrahydrofuran, Diethylether und/oder einem $C_{2-4}$-Alkylalkohol durchführen.

Aus dem erhaltenen 4-Hydroxydervat der Formel XIX kann in einem aprotischen Lösungsmittel, z.B. Diethylether, mit einem Alkalihydrid, beispielsweise Natriumhydrid, das 4-Alkoholat der Verbindung XIX hergestellt werden, welches in der anschließenden Reaktion mit einer Verbindung der Formel III bei Temperaturen zwischen 40° C und 100° C zu einer Verbindung der Formel I, in der
die Gruppierung

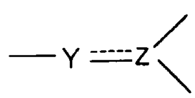 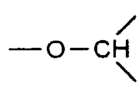

ist,
umgesetzt wird.

Eine weitere Möglichkeit aus einer Verbindung der Formel XIX eine Verbindung der Formel I, in der
die Gruppierung

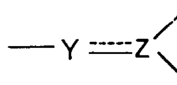 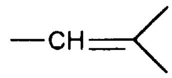

ist,
zu erhalten, besteht in der Durchführung einer Horner-Emmons-Reaktion mit einer Verbindung der Formel XX, in der n vorzugsweise 2 bedeutet, in einem Lösungsmittel, beispielsweise Dimethylformamid, bei Temperaturen zwischen 0° C und 20°C.

Eine gewünschtenfalls anschließende Hydrierungsreaktion einer Verbindung der Formel I, in der die Gruppierung

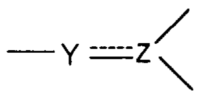 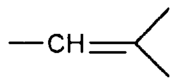

ist,
zu einer Verbindung der Formel I, in der die Gruppierung

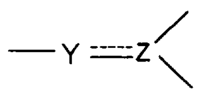 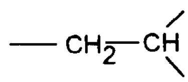

ist,
wird in Gegenwart eines Pt- oder Pd-Katalysators in Essigsäure oder einem geradkettigen oder verzweigten $C_{1-4}$-Alkohol bei 1-100 atm und Temperaturen zwischen 20 °C und 100 °C durchgeführt.

Die erfindungsgemäßen Verbindungen der Formel I liegen als Diastereomere, Enantiomere oder Racemate vor. Die Herstellung von reinen optischen Antipoden aus einem Racemat wird nach an sich bekannten Methoden durchgeführt.

Die erfindungsgemäßen Verbindungen lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in Gegenwart von Wasser in einem der vorgenannten Lösungsmittel.

Die erfindungsgemäßen Verbindungen haben eine ausgeprägte analgetische Wirkung und sind toxikologisch unbedenklich. Sie eignen sich daher als pharmazeutische Wirkstoffe. Erfindungsgegenstand ist dementsprechend auch die Verwendung von 1-Phenyl-2-dimethylaminomethylcyclohexan-1-olverbindungen der Formel I als Wirkstoff in Arzneimitteln, vorzugsweise als Wirkstoff in Schmerzmitteln.

Erfindungsgemäße Arzneimittel enthalten neben mindestens einer 1-Phenyl-2-dimethylaminomethyl-cyclohexan-1-olverbindung der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal, buccal oder lokal, z.B. auf Infektionen an der Haut, an den Schleimhäuten oder an den Augen, appliziert werden soll. Für die orale Applikation eignen sich Zubereitungen in Form von Tabletten, Dragees, Kapseln, Granulaten, Tropfen, Säften und Sirupen, für die parentale, topische und inhalative Applikation Lösungen, Suspensionen, leicht rekonstituierbare Trockenzubereitungen sowie Sprays. Erfindungsgemäße Verbindungen in einem Depot in gelöster Form oder in einem Pflaster, gegebenenfalls unter Zusatz von die Hautpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die intravenöse Gabe der erfindungsgemäßen Arzneimittel ist die bevorzugte Applikationsform.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden Dosierungen im Bereich von 1 bis 200 mg wenigstens einer 1-Phenyl-2-dimethylaminomethyl-cyclohexan-1-olverbindung der Formel I appliziert.

## Beispiele

Soweit nicht anders angegeben , wurde Petrolether mit dem Siedebereich 50° C - 70° C benutzt. Die Angabe Ether bedeutet Diethylether.
Als stationäre Phase für die Säulenchromatographie wurde Kieselgel 60 (0,040 - 0,0063 mm) der Firma E. Merck, Darmstadt, eingesetzt.
Die Mischungsverhältnisse der Elutionsmittel für die chromatographischen Methoden sind stets in Volumen/Volumen angegeben.
Racemattrennungen wurden auf einer Chiracel OD Säule der Firma Daicel Chemical Industries, LTD durchgeführt
Schmp. bedeutet Schmelzpunkt, Zers. Zersetzung und Bsp. Beispiel

### Beispiel 1:

(1RS, 2RS, 4SR)-4-Benzyloxy-2-dimethylaminomethyl-1-(3-methoxy-phenyl)-cyclohexanol, Hydrochlorid (1)

2,42 g Magnesiumspäne (100 mmol) wurden in 25 ml Tetrahydrofuran gerührt und 12,7 ml (100 mmol) 1-Brom-3-methoxy-benzol, gelöst in 64 ml Tetrahydrofuran, zugetropft. Es wurde 1 Stunde unter Rückfluß gekocht, dann auf 5° C - 10° C abgekühlt. Anschließend wurden bei dieser Temperatur 13 g (50 mmol) 4-Benzyloxy-2-dimethylaminomethyl-cyclohexanon (J.R. Hwu et al., J. Chem. Soc., Chem. Commun., 1984, 721-723), gelöst in 65 ml Tetrahydrofuran, zugetropft. Es wurde 1 Stunde bei Raumtemperatur gerührt, auf 5° C - 10° C abgekühlt und die Grignard-Lösung durch Zugabe von 110 ml 20 %iger Ammoniumchlorid-Lösung zersetzt. Die Reaktionsmischung wurde mit 180 ml Ether verdünnt. Anschließend wurden die Phasen getrennt. Die wäßrige Phase wurde zweimal mit 180 ml Ether extrahiert, über Natriumsulfat getrocknet und das Lösungsmittel destillativ entfernt. Der Rückstand (25 g) wurde auf eine 6 x 30 cm Säule, gefüllt mit Kieselgel, gegeben und zuerst mit Ether/n-Hexan 1:1, dann mit Ether/n-Hexan 3:1 eluiert. Man erhielt 10,9 g reine Base, die in Ether/2-Butanon aufgenommen und mit Trimethylchlorsilan/Wasser versetzt wurden. Es wurden 10,6 g kristallines Hydrochlorid (1) erhalten.

Ausbeute: 53 % der Theorie
Schmelzpunkt: 156° C - 158° C

### Beispiel 2:

Die Enantiomeren von (1) :

(1S, 2S, 4R)-4-Benzyloxy-2-dimethylaminomethyl-1-(3-methoxy-phenyl)-cyclohexanol, Hydrochlorid [(-)1]
und

(1R, 2R, 4S)-4-Benzyloxy-2-dimethylaminomethyl-1-(3-methoxy-phenyl)-cyclohexanol, Hydrochlorid [(+)1]

Es wurde aus der Verbindung (1) mit Dichlormethan/Natronlauge die Base freigesetzt, die Lösung getrocknet und Dichlormethan im Vakuum abdestilliert. Das Racemat wurde dann auf einer chiralen HPLC-Säule getrennt. Man erhielt die Basen von [(-)1] und [(+)1], die in 2-Butanon aufgenommen und mit Trimethylchlorsilan/Wasser versetzt wurden. Es wurden die Hydrochloride erhalten.

| | |
|---|---|
| [(-)1]: | Ausbeute: 42,8 % der Theorie |
| | Schmelzpunkt: 212° C - 214° C |
| | $[\alpha]_D^{RT}$ = -20,5° (Wasser, c = 1) |
| [(+)1] | Ausbeute: 40 % der Theorie |
| | Schmelzpunkt: 213° C - 215° C |
| | $[\alpha]_D^{RT}$ = 21,8° (Wasser, c = 1) |

### Beispiel 3:

(1RS, 2RS, 4SR)-4-Benzyloxy-2-dimethylaminomethyl-1-(methoxy-phenyl)-cyclohexanol, Hydrochlorid (2)

1-Brom-3-ethoxy-benzol wurde mit 4-Benzyloxy-2-dimethylaminomethyl-cyclohexanon entsprechend den in Beispiel 1 beschriebenen Bedingungen umgesetzt. Die erhaltene Base wurde über eine Kieselgelsäule mit Ether/Methanol 6:1 gereinigt, anschließend in 2-Butanon aufgenommen und mit Trimethylchlorsilan/Wasser versetzt. Es wurde Verbindung (2) in einer Ausbeute von 43 % der Theorie erhalten.

Schmelzpunkt: 205° C - 207° C.

**Beispiel 4:**

(1RS, 2RS, 4SR)-4-Benzyloxy-2-dimethylaminomethyl-1-(3-isopropoxy-phenyl)-cyclohexanol, Hydrochlorid (3)

1-Brom-3-isopropoxy-benzol wurde mit 4-Benzyloxy-2-dimethylaminomethyl-cyclohexanon entsprechend den in Beispiel 1 beschriebenen Bedingungen umgesetzt. Die erhaltene Base wurde über eine Kieselgelsäule mit Ether/Methanol 6:1 gereinigt, in 2-Butanon/Ether aufgenommen und mit Trimethylchlorsilan/Wasser versetzt. Es wurde Verbindung (3) in einer Ausbeute von 35 % der Theorie erhalten.

Schmelzpunkt: 166° C - 167° C

**Beispiel 5:**

(1RS, 2RS, 4SR)-4-Benzyloxy-2-dimethylaminomethyl-1-[3-(2-methyl-allyloxy)-phenyl]-cyclohexanol, Hydrochlorid (4)

1-Brom-3-(2-methyl-allyloxy)-benzol, hergestellt durch Alkylierung von 1-Brom-3-hydroxy-benzol mit 3-Chlor-2-methyl-1-propen, wurde mit 4-Benzyloxy-2-dimethylaminomethyl-cyclohexanon entsprechend den in Beispiel 1 beschriebenen Bedingungen umgesetzt. Die erhaltene Base wurde über eine Kieselgelsäule mit Ether/Methanol 9:1 gereinigt, in Ether aufgenommen und mit Trimethylchlorsilan/Wasser versetzt. Es wurde Verbindung (4) erhalten.

Ausbeute: 33 % der Theorie
Schmelzpunkt: 166° C - 167° C

**Beispiel 6:**

(1RS, 2RS, 4SR)-3-(4-Benzyloxy-2-dimethylaminomethyl-1-hydroxy-cyclohexyl)-phenol, Hydrochlorid (5)

(3-Brom-phenoxy)-tert-butyl-dimethyl-silan wurde mit 4-Benzyloxy-2-dimethylaminomethyl-cyclohexanon entsprechend den in Beispiel 1 beschriebenen Bedingungen umgesetzt. Die erhaltene Base wurde über eine Kieselgelsäule mit Essigester gereinigt. Anschließend wurde die Silyl-Schutzgruppe mit verdünnter Salzsäure in Tetrahydrofuran abgespalten und das Produkt über eine Kieselgelsäule mit Essigester/Methanol 2:1 gereinigt. Anschließend wurde in Tetrahydrofuran aufgenommem und mit konzentierter Salzsäure versetzt. Verbindung (5) wurde in einer Gesamtausbeute von 47 % der Theorie erhalten.

Schmelzpunkt: 245° C - 247° C (Zers)

**Beispiel 7:**

(1RS, 2RS, 4SR)-4-Benzyloxy-2-dimethylaminomethyl-1-(3-methyl-sulfanyl-phenyl)-cyclohexanol, Hydrochlorid (6)

1-Brom-3-methylsulfanyl-benzol wurde mit 4-Benzyloxy-2-dimethylaminomethyl-cyclchexanon entsprechend den in Beispiel 1 beschriebenen Bedingungen umgesetzt. In Abweichung zu den Bedingungen in Beispiel 1 wurde Ether als Lösungsmittel benutzt und zur Erhöhung der Ausbeute dem Ansatz 1,2-Dibromethan zugegeben. Die erhaltene Base wurde über eine Kieselgelsäule mit n-Hexan, Diisopropylether und Ether gereinigt, in 2-Butanon/Ether aufgenommen und mit Trimethylchlorsilan/Wasser versetzt. Es wurde die kristalline Verbindung (6) in einer Ausbeute von 43 % der Theorie erhalten.

Schmelzpunkt: 194° C - 198° C (Zers.)

**Beispiel 8:**

Die Enantiomeren von (6):

(1S, 2S, 4R)-4-Benzyloxy-2-[(dimethylamino)methyl]-1-(3-methyl-sulfanyl-phenyl)-cyclohexanol, Hydrochlorid [(-)6)] und

(1R, 2R, 4S)-4-Benzyloxy-2-[(dimethylamino)methyl]-1-(3-methyl-sulfanyl-phenyl)-cyclohexanol, Hydrochlorid [(+)6]

Aus der Verbindung (6) wurde mit Methylenchlorid/wäßriger Natriumhydrogencarbonat-Lösung die Base freigesetzt, die Lösung getrocknet und Methylenchlorid im Vakuum abdestilliert. Das Racemat wurde dann auf einer chiralen HPLC-Säule getrennt. Man erhielt die Basen der Verbindungen [(-)6] und [(+)6], die in 2-Butanon und geringen Mengen Diisopropylether aufgenommen und mit Trimethylchlorsilan/Wasser versetzt wurden. Es wurdendie Hydrochloride erhalten.

| | |
|---|---|
| [(-)6]: | Ausbeute: 57 % der Theorie<br>Schmelzpunkt: 195° C - 196° C<br>$[\alpha]_D^{RT}$ = -19° (Wasser, c = 1) |
| [(+)6]: | Ausbeute: 50 % der Theorie<br>Schmelzpunkt: 194° C - 194,5° C<br>$[\alpha]_D^{RT}$ = 20° (Wasser, c = 1) |

**Beispiel 9:**

(1RS, 2RS, 4SR)-2-Dimethylaminomethyl-4-(3-methoxybenzyloxy)-1-(3-methoxy-phenyl)-cyclohexanol,Hydrochlorid (7)

1. Stufe:

4-(3-Methoxybenzyloxy)-cyclohexanon (8)

4,4 g 60 %iges Natriumhydrid in Mineralöl (0,11 mol) wurden in 35 ml absolutem Dimethylformamid unter Stickstoffatmosphäre gerührt. Es wurden 15,7 g (0,1 mol) 1,4-Dioxa-spiro[4.5]decan-8-ol in 65 ml Dimethylformamid gelöst. Diese Lösung wurde zu der Natriumhydridsuspension getropft. Anschließend wurden 16 ml 1-Chlormethyl-3-methoxy-benzol (0,11 mol), gelöst in 25 ml Dimethylformamid, zugegeben. Es wurde 30 Minuten bei 60° C gerührt, auf Eis gegeben, mit Ether extrahiert und über Natriumsulfat getrocknet. Nach Verdampfen des Lösungsmittel wurden 29 g Rohprodukt erhalten. Zur Acetalspaltung wurde 1 Stunde mit einem Gemisch aus 190 ml Tetrahydrofuran und 50 ml konzentrierter Salzsäure gerührt. Es wurde mit gesättigter Natriumchloridlösung verdünnt, die Phasen getrennt, mit Ether extrahiert und getrocknet. Nach destillativer Entfernung des Lösungsmittel wurde die erhaltene Verbindung 8 über eine Kieselgelsäule mit Diisopropylether gereinigt.

Ausbeute 14 g (60 % der Theorie)

2. Stufe:

2-Dimethylaminomethyl-4-(3-methoxybenzyloxy)-cyclohexanon (9)

11,8 g (50 mmol) der Verbindung (8), 0,84 g (28 mmol) Paraformaldehyd und 2,26 g (28 mmol) Dimethylamin, Hydrochlorid wurden in 20 ml Essigsäure gelöst und 15 Minuten in einem Bad von 105° C gerührt. Nach Abdampfen des Lösungsmittel wurde mit Natronlauge ein alkalischer pH-Wert eingestellt und die Mannich-Base mit Dichlormethan extrahiert. Die Lösung wurde getrocknet und Lösungsmittel abdestilliert. Es wurden 12,1 g, 80 % der Theorie, der Verbindung (9) erhalten.

3. Stufe:

(1RS, 2RS, 4SR)-2-Dimethylaminomethyl-4-(3-methoxybenzyloxy)-1-(3-methoxy-phenyl)-cyclohexanol,Hydrochlorid (7)

Die Mannich-Verbindung (9) wurde mit 1-Brom-3-methoxybenzol entsprechend den in Beispiel 1 beschriebenen Bedingungen umgesetzt. Das erhaltene Basengemisch (Substituent in 4-Stellung cis und trans zu OH) wurde auf eine Kieselgelsäule gegeben und nacheinander mit Diisopropylether, Ether und Essigester/ Methanol eluiert. Anschließend wurden beide Isomere über eine Kieselgelsäule mit Ether/Methanol 7:1 gereinigt, in 2-Butanon aufgenommen und mit Trimethylchlorsilan/Wasser, gegebenenfalls unter Zusatz von Ether, versetzt. Es wurden die cis-Form der Verbindung (7) in einer Ausbeute von 7,5 %, Schmelzpunkt 151° C - 153° C und die trans-Form der Verbindung (7) in einer Ausbeute von 20 % der Theorie, Schmelzpunkt 133° C - 135° C erhalten.

**Beispiel 10:**

(1RS, 2RS, 4SR)-4-Allyloxy-2-Dimethylaminomethyl-1-(3-methoxyphenyl)-cyclohexanol, Hydrochlorid (10)

1. Stufe:

8-Allyloxy-1,4-dioxa-spiro[4.5]decan (11)

23,7 g (150 mmol) 1,4-Dioxa-spiro[4.5]decan-8-ol wurden in 120 ml absolutem Dimethylformamid unter Stickstoffatmosphäre gelöst. Nach Zugabe von 7,9 g 50 %igem Natriumhydrid in Mineralöl (165 mmol) wurde bei 20° C 1 Stunde gerührt. Nach Zugabe von 14,3 ml Allylbromid (165 mmol) wurde auf 70° C erwärmt und 1 Stunde gerührt. Danach wurde mit 160 ml Wasser versetzt und bei 10° C - 15° C dreimal mit Ether extrahiert, einmal mit Wasser und einmal mit gesättigter Natriumchloridlösung gewaschen und über Natriumsulfat getrocknet. Nach destillativer Entfernung des Lösungsmittels wurden 24 g Rohprodukt (81 % der Theorie) erhalten.

2. Stufe:

4-Allyloxy-cyclohexanon (12)

19,8 g der Verbindung (11) (0,1 mol) wurden mit 120 ml Ether und 40 ml 6 N Salzsäure 2 Stunden bei Raumtemperatur gerührt. Dann wurde mit Natriumhydrogencarbonat neutralisiert, dreimal mit Ether extrahiert und die Etherlösung über Natriumsulfat getrocknet. Nach destillativer Entfernung des Lösungsmittels wurden 15,2 g Rohprdukt erhalten, die über eine Kieselgelsäule mit Diisopropylether/n-Hexan 3:1 gereinigt wurde.

Ausbeute: 12,8 g (83 % der Theorie)

3. Stufe:

4-Allyloxy-2-dimethylaminomethyl-cyclohexanon, Hydrochlorid (13)

15,5 g der Verbindung (120 (100 mmol), 1,5 g (50 mmol) Paraformaldehyd und 4,1 g (50 mmol) Dimethylamin, Hydrochlorid wurden in 30 ml Essigsäure 25 Minuten in einem Bad von 105° C gerührt. Essigsäure wurde im Vakuum abdestilliert, der Rückstand in 110 ml 2-Butanon gelöst. Es wurde Verbindung (13) in einer Ausbeute von 77 % der Theorie, Schmelzpunkt 125° C - 127° C, erhalten.

4. Stufe:

(1RS, 2RS, 4SR)-4-Allyloxy-2-Dimethylaminomethyl]-1-(3-methoxyphenyl)-cyclohexanol, Hydrochlorid (10)

Die Base (13) wurde mit 1-Bromo-3-methoxy-Benzol entsprechend den in Beispiel 1 beschriebenen Bedingungen umgesetzt. Die erhaltene Base wurde über eine Kieselgelsäule mit Ether/Diisopropylether 1:4 gereinigt und in 2-Butanon/Ether 1:1 aufgenommen. Nach Zugabe von Trimethylchlorsilan/Wasser wurde die Verbindung (10) in einer Ausbeute von 37 % der Theorie erhalten.

Schmelzpunkt: 88° C - 94° C .

**Beispiel 11:**

(1RS, 2RS, 4SR)-2-Dimethylaminomethyl-1-(3-methoxy-phenyl)-4-(2-methyl-2-allyloxy)-cyclohexanol, Hydrochlorid (14)

1. Stufe:

8-(2-Methyl-allyloxy)-1,4-dioxa-spiro[4.5]decan (15)

Die Umsetzung zur Verbindung (15) wurde mit 1,4-Dioxa-spiro[4.5]decan-8-ol und 2-Methyl-allylbromid entsprechend den unter Beispiel 10 Stufe 1 beschriebenen Bedingungen durchgeführt. Die erhaltene Verbindung (15) wurde über eine Kieselgelsäule mit Diisopropylether/n-Hexan 1:2 gereinigt. Es wurde die Verbindung (15) als helles Öl in einer Ausbeute von 81 % der Theorie erhalten.

2. Stufe:

4-(2-Methyl-allyloxy)-cyclohexanon (16)

Die Acetalspaltung von Verbindung (15) wurde entsprechend den unter Beispiel 10 Stufe 2 beschriebenen Bedingungen durchgeführt. Die erhaltene Verbindung (16) wurde über eine Kieselgelsäule mit Diisopropylether/n-Hexan 2:1 gereinigt. Es wurde Verbindung (16) als Öl in einer Ausbeute von 62 % der Theorie erhalten.

3. Stufe:

2-Dimethylaminomethyl- 4-(2-methyl-2-allyloxy) cyclohexanon Hydrochlorid (17)

Die Mannichreaktion wurde mit Verbindung (16) und Dimethylamin, Hydrochlorid entsprechend den in Beispiel 10 Stufe 3 beschriebenen Bedingungen durchgeführt. Man erhielt aus 2-Butanon die Verbindung (17) in einer Ausbeute von 38 % der Theorie.

Schmelzpunkt: 111° C - 112° C.

4. Stufe:

(1RS, 2RS, 4SR)-2-Dimethylaminomethyl-1-(3-methoxy-phenyl)-4-(2-methyl-2-allyloxy)-cyclohexanol, Hydrochlorid (14)

Verbindung (17) wurde mit 1-Bromo-3-methoxy-benzol entsprechend den in Beispiel 1 beschriebenen Bedingungen umgesetzt. Die erhaltene Base wurde über eine Kieselgelsäule mit Essigester/Methanol 1:1 gereinigt und in Ether aufgenommen. Nach Zugabe von Trimethylchlorsilan/Wasser wurde Verbindung (14) in einer Ausbeute von 36 % der Theorie erhalten.

Schmelzpunkt: 112° C - 114 °C

**Beispiel 12:**

(1RS, 2RS, 4SR)-2-Dimethylaminomethyl-4-(2-methyl-2-allyloxy)-1-[3-(2-methyl-2-allyloxy)-phenyl]-cyclohexanol, Hydrochlorid (18)

Die Base (17) und 1-Bromo-3-(2-methyl-allyloxy)-benzol wurden entsprechend den in Beispiel 1 beschriebenen Bedingungen umgesetzt. Das Rohprodukt wurde auf eine Kieselgelsäule gegeben und mit Diisopropylether/Ether eluiert. Das erhaltene bezüglich Position 1 epimere Gemisch wurde durch HPLC in die cis- und trans-Diastereomeren getrennt und anschließend in 2-Butanon/Diisopropylether aufgenommen. Nach Zugabe von Trimethylchlorsilan/Wasser wurde Verbindung (18) in einer Ausbeute von 18 % der Theorie erhalten.

Schmelzpunkt: 151° C - 152,5° C.

**Beispiel 13:**

(1RS, 2RS, 4SR)-2-Dimethylaminomethyl-4-(2-methyl-2-allyloxy)-1-(3-methyl-sulfanyl-phenyl)-cyclohexanol, Hydrochlorid (19)

Die Base der Verbindung (17) und 1-Bromo-3-methylsulfanyl-benzol wurden entsprechend den in Beispiel 1 beschriebenen Bedingungen umgesetzt. Abweichend von den Bedingungen in Beispiel 1 wurde als Lösungsmittel Ether benutzt und zur Erhöhung der Ausbeute wurde 1,2-Dibromethan dem Reaktionsansatz zugegeben. Das erhaltene Rohprodukt wurde über eine Kieselgelsäule mit Diisopropylether/Ether 1:1 gereinigt und in Essigester aufgenommen. Nach Zugabe von Trimethylchlorsilan/Wasser wurde Verbindung (19) in einer Ausbeute von 40 % der Theorie erhalten.

Schmelzpunkt: 146° C - 150° C

**Beispiel 14:**

(1RS, 2RS, 4SR)-2-Dimethylaminomethyl-4-isobutoxy-1-(3-methoxy-phenyl)-cyclohexanol, Hydrochlorid (20)

1. Stufe:

4-Isobutoxy-cyclohexanon (21)

16,9 g (0,1 mol) der Verbindung (16) wurden in 90 ml Methanol gelöst. Nach Zugabe von 1,8 g Palladium-Kohle (10 % Pd-Gehalt) wurde bei Raumtemperatur und Normaldruck hydriert. Anschließend wurde der Katalysator entfernt und das Lösungsmittel im Vakuum abdestilliert. Die erhaltenen 15 g Rohprodukt wurden mit 60 ml Ether und 30 ml 4 N Salzsäure 1 Stunde bei Raumtemperatur gerührt. Anschließend wurde mit Natriumhydrogencarbonat neutralisiert, die Etherphase abgetrennt und zweimal mit Ether extrahiert. Die etherische Lösung wurde über Natriumsulfat getrocknet und Ether abdestilliert. Die erhaltenen 10,5 g Öl wurden über eine Kieselgelsäule mit Diisopropylether/n-Hexan 1:1 gereinigt. Es wurden 6,85 g Verbindung (21) in einer Ausbeute von 40 % der Theorie erhalten.

2. Stufe:

2-Dimethylaminomethyl-4-isobutoxy-cyclohexanon, Hydrochlorid (22)

Die Verbindung (21) wurde mit Dimethylamin, Hydrochlorid entsprechend den in Beispiel 10 Stufe 3 beschriebenen Bedingungen umgesetzt. Es wurde aus 2-Butanon die Verbindung der Formel (22) in einer Ausbeute von 53 % der Theorie erhalten.

Schmelzpunkt: 113° C - 115° C

3. Stufe:

(1RS, 2RS, 4SR)-2-Dimethylaminomethyl-4-isobutoxy-1-(3-methoxy-phenyl)-cyclohexanol, Hydrochlorid (20)

Die Base der Verbindung (22) wurde mit 1-Brom-3-methoxy-benzol entsprechend den in Beispiel 1 beschriebenen Bedingungen umgesetzt. Die erhaltene Base wurde über eine Kieselgelsäule mit Essigester/ Methanol 1:1 gereinigt und in Ether aufgenommen. Nach Zugabe von Trimethylchlorsilan/Wasser wurde Verbindung (20) in einer Ausbeute von 38 % der Theorie erhalten.

Schmelzpunkt: 112° C - 116° C

**Beispiel 15:**

(1RS, 2RS, 4SR)-2-Dimethylaminomethyl-4-isobutoxy-(3-methylsulfanyl-phenyl)-cyclohexanol, Hydrochlorid (23)

Verbindung (22) wurde mit 1-Brom-3-methylsulfanyl-benzol entsprechend den in Beispiel 1 beschriebenen Bedingungen umgesetzt. Abweichend von den in Beispiel 1 beschriebenen Bedingungen wurde Ether als Lösungsmittel benutzt und zur Erhöhung der Ausbeute 1,2-Dibromethan zugegeben. Das erhaltene Rohprodukt wurde über

eine Kieselgelsäule mit Diisopropylether gereinigt und in Tetrahydrofuran/Ether aufgenommen. Die nach Zugabe von Trimethylchlorsilan/Wasser erhaltenen Kristalle wurden aus Essigester umkristallisiert. Verbindung (23) wurde in einer Ausbeute von 32 % der Theorie erhalten.

Schmelzpunkt: 120° C - 122° C .

**Beispiel 16:**

(1RS, 2RS, 4SR)-2-Dimethylaminomethyl-1-(3-methoxy-phenyl)-4-phenoxy-cyclohexanol, Hydrochlorid (24)

1. Stufe:

8-Phenoxy-1,4-dioxa-spiro[4.5]decan (25)

440 mg 60 %iges Natriumhydrid in Mineralöl (0,12 mmol) wurden in 9 ml absolutem Dimethylformamid unter Stickstoffatmosphäre gerührt. Es wurden 1,1 g (0,12 mmol) Phenol und anschließend 3,1 g (10 mmol) Toluol-4-sulfonsäure-1,4-dioxa-spiro[4.5]dec-8-ylester (Gray et al., J. Org. Chem., 35, (1970), 1525-1533), gelöst in 6 ml Dimethylformamid, zugegeben. Es wurde 2 Stunden bei einer Temperatur zwischen 80° C und 85° C gerührt. Nach dem Abkühlen wurde der Ansatz auf Eis gegeben, mit Ether extrahiert, die Lösung mit verdünnter Natronlauge gewaschen und mit Natriumsulfat getrocknet. Nach Abdampfen des Lösungsmittels wurde das Rohprodukt über eine Kieselgelsäule mit Diisopropylether gereinigt. Es wurden 1,27 g (54 % der Theorie) Verbindung (25) als Öl erhalten.

2. Stufe:

4-Phenoxy-cyclohexanon (26)

11,7 g (50 mmol) 8-Phenoxy-1,4-dioxa-spiro[4.5]decan (25) wurden in 250 ml Ether gelöst. Unter Rühren wurden 50 ml Wasser und 37,5 ml konzentrierte Salzsäure zugegeben. Es wurde 2 Stunden gerührt und über Nacht stehengelassen. Nach anschließender Phasentrennung wurde mit Ether extrahiert, mit gesättigter Kochsalzlösung gewaschen, über Natriumsulfat getrocknet und das Lösungsmittel abdestilliert. Es wurden 9 g (91 % der Theorie) Verbindung (26) erhalten.

3. Stufe:

2-Dimethylaminomethyl-4-phenoxy-cyclohexanon, Hydrochlorid (27)

9,5 g (50 mmol) 4-Phenoxy-cyclohexanon (26), 0,765 g (25 mmol) Paraformaldehyd und 2,08 g (25 mmol) Dimethylamin, Hydrochlorid wurden in 17 ml Essigsäure 20 Minuten in einem Ölbad mit einer Temperatur von 105° C erwärmt. Danach wurde Lösungsmittel im Vakuum abdestilliert, der Rückstand zweimal mit 2-Butanon versetzt und anschließend das 2-Butanon im Vakuum destillativ entfernt. Das erhaltene Salz wurde in 30 ml 2-Butanon aufgenommen. Es wurden 2,85 g Verbindung (27) (40 % der Theorie) erhalten.

Schmelzpunkt 104° C-106° C (40 % der Theorie).

4. Stufe:

(1RS, 2RS, 4SR)-2-Dimethylaminomethyl-1-(3-methoxy-phenyl)-4-phenoxy-cyclohexanol, Hydrochlorid (24)

Die Base der Verbindung (27) wurde mit 1-Brom-3-methoxy-benzol entsprechend den in Beispiel 1 beschriebenen Bedingungen umgesetzt. Die erhaltene Base wurde über eine Kieselgelsäule mit Essigester/Methanol 6:1 gereinigt und in 2-Butanon aufgenommen. Nach Zugabe von Trimethylchlorsilan/Wasser wurde Verbindung (24) in einer Ausbeute von 47 % der Theorie erhalten.

Schmelzpunkt: 216° C - 218° C

**Beispiel 17:**

(1RS, 2RS, 4SR)-2-Dimethylaminomethyl-1-(3-ethoxy-phenyl)-4-phenoxy-cyclohexanol, Hydrochlorid (28)

Die Base der Verbindung (27) wurde mit 1-Brom-3-ethoxybenzol entsprechend den in Beispiel 1 beschriebenen Bedingungen umgesetzt. Die erhaltene Base wurde über eine Kieselgelsäule mit Essigester/Methanol 6:1 gereinigt und in 2-Butanon aufgenommen. Nach Zugabe von Trimethylchlorsilan/Wasser wurde Verbindung (28) in einer Ausbeute von 49 % der Theorie erhalten.

Schmelzpunkt: 237° C - 239° C

**Beispiel 18:**

(1RS, 2RS, 4SR)-2-Dimethylaminomethyl-1-(3-isopropoxyphenyl)-4-phenoxy-cyclohexanol, Hydrochlorid (29)

Die Base der Verbindung (27) wurde mit 1-Brom-3-propoxy-benzol entsprechend den in Beispiel 1 beschriebenen Bedingungen umgesetzt. Die erhaltene Base wurde über eine Kieselgelsäule mit Essigester/Methanol 6:1 gereinigt und in 2-Butanon aufgenommen. Nach Zugabe von Trimethylchlorsilan/Wasser wurde Verbindung (29) in einer Ausbeute von 38 % der Theorie erhalten.

Schmelzpunkt: 200° C - 202° C

**Beispiel 19:**

(1RS, 2RS, 4SR)-3-(2-Dimethylaminomethyl)-1-hydroxy-4-phenoxy-cycohexyl)-phenol, Hydrochlorid (30)

Die Base der Verbindung (27) wurde mit 1-Brom-3-benzyloxy-benzol entsprechend den in Beispiel 1 beschriebenen Bedingungen umgesetzt. Es wurde das mit einer Benzylgruppe geschützte Phenol in einer Ausbeute von ca. 50 % der Theorie (Rohprodukt) erhalten. Anschließend wurde in Methanol aufgenommen, Palladium-Kohle (10 % Pd-Gehalt) zugegeben und die Benzylgruppe durch Hydrierung bei Raumtemperatur unter Normaldruck abgespalten. Die erhaltene Base wurde über eine Kieselgelsäule mit Essigester/Methanol 6:1 gereinigt und in 2-Butanon aufgenommen. Nach Zugabe von Trimethylchlorsilan/Wasser wurde Verbindung (30) in einer Ausbeute von 50 % der Theorie erhalten.

Schmelzpunkt: 220° C - 222° C

**Beispiel 20:**

(1RS, 2RS, 4SR)-2-Dimethylaminomethyl-1-[3-(2-methylallyloxy)-phenyl]-4-phenoxy-cyclohexanol, Hydrochlorid (31)

Die Base der Verbindung (27) wurde mit 1-Brom-3-(2-methyl-allyloxy)-benzol entsprechend den in Beispiel 1 beschriebenen Bedingungen umgesetzt. Die erhaltene Base wurde über eine Kieselgelsäule mit Ether gereinigt und in 2-Butanon aufgenommen. Nach Zugabe von Trimethylchlorsilan/Wasser wurde Verbindung (31) in einer Ausbeute von 51 % der Theorie erhalten.

Schmelzpunkt: 193° C - 195° C

**Beispiel 21:**

(1RS, 2RS, 4SR)-2-Dimethylaminomethyl-4-(3-methoxyphenoxy)-1-(3-methoxy-phenyl)-cyclohexanol, Hydrochlorid (32)

1. Stufe:

4-(3-Methoxy-phenoxy)-cyclohexanon (33)

3-Methoxy-phenol wurde mit Toluol-4-sulfonsäure-1,4-dioxa-spiro[4.5]dec-8-ylester entsprechend den in Beispiel 16 Stufe 1 beschriebenen Bedingungen umgesetzt. Das Rohprodukt wurde ohne Reinigung einer Acetalspaltung entsprechend den in Beispiel 16 Stufe 2 beschriebenen Bedingungen unterzogen. Das erhaltene Keton wurde auf

einer Kieselgelsäule mit Diisopropylether gereinigt. Verbindung (33) wurde in einer Ausbeute von 57 der Theorie erhalten.

2. Stufe:

2-Dimethylaminomethyl-4-(3-methoxy-phenoxy)-cyclohexanon (34)

Die Verbindung (33) wurde mit Diethylamin, Hydrochlorid entsprechend den in Beispiel 16 Stufe 3 beschriebenen Bedingungen umgesetzt. Zur Aufarbeitung wurde Essigsäure abdestilliert, der Rückstand in Wasser gelöst und mit Ether extrahiert. Der pH-Wert der wässrigen Phase wurde mit Natronlauge alkalisch eingestellt und das Produkt mit Dichlormethan extrahiert. Das Lösungsmittel wurde destillativ entfernt und Verbindung (34) als cis-trans-Gemisch in einer Ausbeute von 50 % der Theorie erhalten.

3. Stufe:

(1RS, 2RS, 4SR)-2-Dimethylaminomethyl-4-(3-methoxyphenoxy)-1-(3-methoxy-phenyl)-cyclohexanol, Hydrochlorid (32)

Verbindung (34) wurde mit 1-Brom-3-methoxy-benzol entsprechend den in Beispiel 1 beschriebenen Bedingungen umgesetzt. Das bezüglich Position 1 epimere Basengemisch wurde auf einer Kieselgelsäule nacheinander mit Diisopropylether und Essigester eluiert. Die Fraktion, die mit der cis-Form angereichert war, wurde dann über eine Kieselgelsäule mit Ether/Methanol 7:1 gereinigt. Anschließend wurde in 2-Butanon aufgenommen und Trimethylchlorsilan/Wasser zugegeben. Verbindung (32) (Position 4 cis zu OH) wurde in einer Ausbeute von 10 % der Theorie erhalten.

Schmelzpunkt: 208° C - 210° C .

**Beispiel 22:**

(1RS, 2RS, 4SR)-4-Benzyl-2-dimethylaminomethyl-1-(3-methoxy-phenyl)-cyclohexanol, Hydrochlorid (35)

1. Stufe:

4-Benzyl-2-dimethylaminomethyl-cyclohexanon, Hydrochlorid (36)

4-Benzylcyclohexanon wurde mit Dimethylamin, Hydrochlorid entsprechend den in Beispiel 10 Stufe 3 beschriebenen Bedingungen umgesetzt. Verbindung (36) wurde in kristalliner Form in einer Ausbeute von 50 % der Theorie erhalten.

Schmelzpunkt: 136° C - 138° C

2. Stufe:

(1RS, 2RS, 4SR)-4-Benzyl-2-dimethylaminomethyl-1-(3-methoxy-phenyl)-cyclohexanol, Hydrochlorid (35)

Die Base der Verbindung (36) wurde mit 1-Brom-3-methoxy-benzol entsprechend den in Beispiel 1 beschriebenen Bedingungen umgesetzt. Die erhaltene Base wurde über eine Kieselgelsäule mit Ether/Methanol 7:1 gereinigt und in Ether aufgenommen. Nach Zugabe von Trimethylchlorsilan/Wasser wurde Verbindung (35) in einer Ausbeute von 55 % der Theorie erhalten.

Schmelzpunkt: 138° C - 142° C

**Beispiel 23:**

(1RS, 2RS, 4SR)-3-(4-Benzyl-2-dimethylaminomethyl-1-hydroxy-cyclohexyl)-phenol, Hydrochlorid (37)

1. Stufe:

4-Benzyl-1-(3-benzyloxyphenyl)-2-dimethylaminomethylcyclohexanol (38)

Die Base der Verbindung (36) wurde mit 1-Brom-3-benzyloxy-benzol entsprechend den in Beispiel 1 beschriebenen Bedingungen umgesetzt. Die erhaltene Base wurde über eine Kieselgelsäule mit Ether/Methanol 7:1 gereinigt.

Ausbeute an Verbindung (38) : 76 % der Theorie

2. Stufe:

(1RS, 2RS, 4SR)-3-(4-Benzyl-2-dimethylaminomethyl-1-hydroxy-cyclohexyl)-phenol, Hydrochlorid (37)

Verbindung (38) wurde in Methanol gelöst. Nach Zugabe von Palladium-Kohle (10 % Pd-Gehalt) wurde bei Raumtemperatur und Normaldruck hydriert. Nach Abfiltrieren des Katalysators und destillativer Entfernung des Lösungsmittels wurde in Ether aufgenommen. Durch anschließende Zugabe von Trimethylchlorsilan/Wasser wurde das Hydrochlorid erhalten. Nach Umkristallisieren aus 2-Butanon/Wasser erhielt man Verbindung (37) in einer Ausbeute von 61 % der Theorie.

Schmelzpunkt: 187° C - 192° C

**Beispiel 24:**

(1RS, 2RS, 4SR)-3-[2-Dimethylaminomethyl-1-hydroxy-4-(4-methyl-benzyl)-cyclohexyl]-phenol, Hydrochlorid (39)

1. Stufe:

4-Hydroxy-4-(4-methyl-benzyl)-cyclohexanon (40)

Zu 2,9 g (120 mmol) Magnesiumspänen wurden geringe Mengen einer Lösung von 22,2 g 1-Bromethyl-4-methyl-benzol (120 mmol) in 80 ml Ether getropft. Nach dem Start der Grignard-Reaktion wurde der Rest der Lösung zugegeben und 30 Minuten unter Rückfluß gekocht. Anschließend wurde auf 0 °C - 10° C abgekühlt und bei dieser Temperatur eine Lösung von 15,7 g (100 mmol) 1,4-Dioxa-spiro[4.5]decan-8-on, gelöst in 35 ml Tetrahydrofuran und 70 ml Ether, zugegeben. Es wurde 2 Stunden unter Rückfluß gekocht. Nach dem Abkühlen auf 0° C - 10° C wurden 80 ml einer 20 %igen Ammoniumchlorid-Lösung zugegeben und die Phasen getrennt. Anschließend wurde mit Ether extrahiert, über Natriumsulfat getrocknet und Lösungsmittel destillativ entfernt. Es wurden 27,5 g Rohprodukt erhalten, die in 300 ml Ether gelöst, mit 200 ml halbkonzentrierter Salzsäure versetzt und 3 Stunden bei Raumtemperatur gerührt wurden. Anschließend wurde mit gesättigter Kochsalzlösung verdünnt. Nach Phasentrennung wurde mit Ether extrahiert und über Natriumsulfat getrocknet. Anschließend wurde Lösungsmittel abdestilliert und über eine Kieselgelsäule mit Ether/Hexan 2:1 gereinigt.

Die Ausbeute an Verbindung (40) betrug 11,5 g (53 % der Theorie).

2. Stufe:

4-(4-Methyl-benzyl)-cyclohexanon (41)

10,9 g (50 mmol) der Verbindung (40) wurden in 250 ml Ameisensäure gelöst und 3 Stunden unter Rückfluß gekocht. Es wurde ein Olefingemisch erhalten. Die Ameisensäure wurde im Vakuum abdestilliert und die Olefine über eine Kieselgelsäule mit Ether/Hexan 1:1 gereinigt. Es wurden 7,5 g Olefingemisch erhalten, das in Ethanol gelöst und nach Zugabe von Palladium-Kohle (10 % Pd-Gehalt) bei Raumtemperatur und Normaldruck hydriert wurde. Nach Abfiltrieren des Katalysators und destillativer Entfernung des Lösungsmittels erhielt man 7,8 g Keton. Die Acetalspaltung und anschließende Aufarbeitung wurde entsprechend den in Stufe 1 beschriebenen Bedingungen durchgeführt. Es wurden 6,7 g Keton erhalten, die über eine Kieselgelsäule mit Ether/Hexan 1:2 gereinigt wurden.

Die Ausbeute der Verbindung (41) betrug 5,15 g, 51 % der Theorie.

3. Stufe:

2-Dimethylaminomethyl-4-(4-methyl-benzyl)-cyclohexanon, Hydrochlorid (42)

Verbindung (41) wurde mit Dimethylamin, Hydrochlorid entsprechend den in Beispiel 10 Stufe 3 beschriebenen Bedingungen umgesetzt. Es wurde Verbindung (42) in kristalliner Form in einer Ausbeute von 46 % der Theorie erhalten.

Schmelzpunkt: 124° C - 127° C

4. Stufe:

1-(3-Benzyloxyphenyl)-2-dimethylaminomethyl-4-(4-methyl-benzyl)-cyclohexanol (43)

1-Brom-3-benzyloxy-benzol und die Base der Verbindung (42) wurden entsprechend den in Beispiel 1 beschriebenen Bedingungen umgesetzt. Das erhaltene Produkt wurde in Ether/Hexan gelöst und die Base mit 10 %iger Essigsäure extrahiert. Nach Einstellen eines alkalischen pH-Wertes wurde die Verbindung (43) mit Ether extrahiert und über Natriumsulfat getrocknet. Nach destillativer Entfernung des Lösungsmittels wurde über eine Aluminiumoxid-Säule (3,6 % Wasser) mit Ether/Disopropylether 1:1 gereinigt. Verbindung (43) wurde in einer Ausbeute von 65 % der Theorie erhalten.

5. Stufe:

(1RS, 2RS, 4SR)-3-[2-Dimethylaminomethyl-1-hydroxy-4-(4-methyl-benzyl)-cyclohexyl]-phenol, Hydrochlorid (39)

Verbindung (43) wurde entsprechend den in Beispiel 23 Stufe 2 beschriebenen Bedingungen hydriert. Anschließend wurde in Dichlormethan/Ether 1:1 aufgenommen und Trimethylchlorsilan/Wasser zugegeben. Nach Umkristallisieren aus 2-Butanol/Wasser wurde Verbindung (39) in einer Ausbeute von 56 % der Theorie erhalten.

Schmelzpunkt: 188° C - 191° C

**Beispiel 25:**

(1RS, 2RS, 4SR)-2-Dimethylaminomethyl-1-(3-methoxy-phenyl)-4-(4-methylbenzyl)-cyclohexanol, Hydrochlorid (44)

Die Base der Verbindung (42) wurde mit 1-Brom-3-methoxy-benzol entsprechend den in Beispiel 1 beschriebenen Bedingungen umgesetzt. Die erhaltene Base wurde über eine Kieselgelsäule mit Ether/Methanol 7:1 gereinigt und in Ether aufgenommen. Nach Zugabe von Trimethylchlorsilan/Wasser wurde Verbindung (44) in einer Ausbeute von 39 % der Theorie erhalten.

Schmelzpunkt: 116° C - 122° C

**Beispiel 26:**

(1RS, 2RS, 4SR)-2-Dimethylaminomethyl-1-(3-methoxy-phenyl)-4-phenylethyl-cyclohexanol, Hydrochlorid (45)

1. Stufe:

4-Hydroxy-4-phenylethyl-cyclohexanon (46)

(2-Chlorethyl)-benzol wurde mit 1,4-Dioxa-spiro[4.5] decan-8-on entsprechend den in Beispiel 24 Stufe 1 beschriebenen Bedingungen umgesetzt. Das Produkt wurde auf einer Kieselgelsäule mit Ether/n-Hexan 5:1 gereinigt. Verbindung (46) wurde in einer Ausbeute von 71 % der Theorie erhalten.

2. Stufe:

4-(Phenethyl)-cyclohexanon (47)

Verbindung (46) wurde entsprechend den in Beispiel 24 Stufe 2 beschriebenen Bedingungen dehydratisiert und hydriert. Nach der Dehydratisierung wurde das erhaltenen Olefingemisch über eine Kieselgelsäule mit Diisopropylether/n-Hexan 3:1 gereinigt. Die nach der Hydrierung erhaltene Verbindung der Formel (47) wurde über eine Kieselgelsäule mit Diisopropylether/n-Hexan 1:1 gereinigt. Verbindung (47) wurde in einer Ausbeute von 54 % der Theorie erhalten.

3. Stufe:

2-Dimethylaminomethyl-4-(phenethyl)-cyclohexanon, Hydrochlorid (48)

20,2 g (0,1 mol) der Verbindung (47), 1,5 g (0,05 mol) Paraformaldehyd und 4,07 g (0,05 mol) Dimethylamin, Hydrochlorid wurden in 40 ml Essigsäure gelöst und unter Rühren 20 Minuten in einem Bad von 105° C erwärmt. Anschließend wurde Essigsäure im Vakuum abdestilliert, der Rückstand in 100 ml Wasser gelöst und mit Ether extrahiert. Die wäßrige Phase wurde mit Natronlauge auf einen pH-Wert von 11 eingegestellt und die Mannich-Base mit Dichlormethan extrahiert. Nach Trocknen und destillativer Entfernung des Lösungsmittels wurden 17,5 g der Base von Verbindung (47) erhalten, die in 2-Butanon aufgenommen wurden. Durch anschließende Zugabe von Trimethylchlorsilan/Wasser wurden 17,2g der Verbindung (48) in einer Ausbeute von 58 % der Theorie erhalten.

Schmelzpunkt: 159° C - 160° C

4. Stufe:

(1RS, 2RS, 4SR)-2-Dimethylaminomethyl-1-(3-methoxy-phenyl)-4-phenylethyl-cyclohexanol, Hydrochlorid (45)

Die Base der Verbindung (48) wurde mit 1-Brom-3-methoxy-benzol entsprechend den in Beispiel 1 beschriebenen Bedingungen umgesetzt. Die erhaltene Base wurde über eine Kieselgelsäule mit Ether/Methanol 7:1 und anschließend über HPLC gereinigt. Es wurde in 2-Butanon aufgenommen und Trimethylchlorsilan/Wasser, gegebenenfalls unter Zusatz von Ether, zugegeben. Verbindung (45) wurde in einer Ausbeute von 40 % der Theorie erhalten.

Schmelzpunkt: 170° C - 173° C

**Beispiel 27:**

(1RS, 2RS, 4SR)-3-(2-Dimethylaminomethyl-1-hydroxy-4-phenethyl-cyclohexyl)-phenol, Hydrochlorid (49)

1. Stufe:

1-(3-Benzyloxyphenyl-2-dimethylaminomethyl-4-phenethyl-cyclohexanol (50)

Die Base der Verbindung (48) wurde mit 1-Bromo-3-benzyloxy-benzol entsprechend den in Beispiel 1 beschriebenen Bedingungen umgesetzt. Die erhaltene Base wurde über eine Kieselgelsäule mit Ether/Methanol 6:1 gereinigt. Verbindung (50) wurde in einer Ausbeute von 87 % der Theorie erhalten.

2. Stufe:

(1RS, 2RS, 4SR)-3-(2-Dimethylaminomethyl-1-hydroxy-4-phenethyl-cyclohexyl)-phenol, Hydrochlorid (49)

Verbindung (50) wurde entsprechend den in Beispiel 23 Stufe 2 beschriebenen Bedingungen hydriert. Die erhaltene Base wurde mit Trimethylchlorsilan/Wasser in das Hydrochlorid umgewandelt. Verbindung (49) wurde in einer Ausbeute von 63 % der Theorie erhalten.

Schmelzpunkt: 254° C - 256° C

**Beispiel 28:**

(1RS, 2RS, 4SR)-2-Dimethylaminomethyl-1-(3-methylsulfanyl-phenyl)-4-phenethyl-1-cyclohexanol, Hydrochlorid (51)

Die Base der Verbindung (48) wurde mit 1-Brom-3-methylsulfanyl-benzol entsprechend den in Beispiel 1 beschriebenen Bedingungen umgesetzt. Abweichend von den in Beispiel 1 beschriebenen Bedingungen wurde Ether als Lösungsmittel benutzt und dem Reaktionsansatz zur Erhöhung der Ausbeute 1,2-Dibromethan zugesetzt. Das Rohprodukt wurde über eine Kieselgelsäule mit Diisopropylether gereinigt und in 2-Butanon aufgenommen. Nach Zugabe von Trimethylchlorsilan/Wasser wurde Verbindung (51) in einer Ausbeute von 23 % der Theorie erhalten.

Schmelzpunkt: 161° C - 163° C

**Beispiel 29:**

(1RS, 2RS, 4SR)-4-(Cyclopentyl-ethyl)-2-dimethylaminomethyl-1-(3-methoxyphenyl)-cyclohexanol, Hydrochlorid (52)

1. Stufe:

(Cyclopentylethyltriphenylphosphonium)bromid (53)

17,7 g (100 mmol) (2-Bromethyl)-cyclopentan, 32,5 g (124 mmol) Triphenylphosphin und 100 ml Toluol wurden 56 Stunden unter Rückfluß gekocht. Es wurde über Nacht bei Raumtemperatur gerührt. Die entstandenen Kristalle wurden abgesaugt, mit Ether gewaschen und im Vakuum getrocknet. Die Ausbeute der Verbindung (53) betrug 35,6 g (82 % der Theorie).

Schmelzpunkt: 210° C - 213° C

2. Stufe:

8-(2-Cyclopentyl-ethyliden)-1,4-dioxa-spiro[4.5]decan (53)

Die Reaktion wurde in einer Stickstoffatmosphäre unter Feuchtigkeitsausschluß durchgeführt. Zu 5,6 g (50 mmol) Kalium-tert-butylat in 400 ml Toluol wurden 21,9 g (50 mmol) Verbindung (52) gegeben. Es wurde 30 Minuten bei Raumtemperatur, 1 Stunde bei 80° C gerührt und anschließend auf 60° C abgekühlt. 7,8 g (50 mmol) 1,4-Dioxa-spiro[4.5]decan-8-on wurden zugegeben. Anschließend wurde 18 Stunden bei 60° C gerührt und abgekühlt. Es wurden 100 ml Wasser hinzugetropft. Die organische Phase wurde abgetrennt, die wäßrige Phase zweimal mit Toluol extrahiert, mit Natriumsulfat getrocknet und das Lösungsmittel destillativ entfernt. Es wurden 29 g Öl erhalten, die auf einer Kieselgelsäule mit Diisoproplether/n-Hexan 1:4 gereinigt wurden.

Die Ausbeute betrug 6,8 g (58 % der Theorie) Verbindung (54).

3. Stufe:

(2-Cyclopentyl-ethyl)-cyclohexanon (55)

9,45 g (40 mmol) der Verbindung (54) wurden in 50 ml Methanol gelöst, mit 1,3 g Palladium-Kohle (10 % Pd-Gehalt) versetzt und bei Normaldruck und Raumtemperatur hydriert. Der Katalysator wurde abgetrennt, das Lösungsmittel destillativ entfernt und der Rückstand mit 10 Teilen Tetrahydrofuran und 4 Teilen halbkonzentrierter Salzsäure gerührt. Anschließend wurde mit Natriumhydrogencarbonat neutralisiert, mit Ether extrahiert, mit Natriumsulfat getrocknet und das Lösungsmittel abdestilliert. Das erhaltene Öl wurde über eine Kieselgelsäule mit Ether/n-Hexan 1:9 gereinigt. Verbindung (55) wurde in einer Ausbeute von 5,9 g (76 % der Theorie) erhalten.

4. Stufe:

4-(2-Cyclopentyl-ethyl)-2-dimethylaminomethyl-cyclohexanon, Hydrochlorid (56)

Verbindung (55) wurde mit Dimethylamin, Hydrochlorid entsprechend den in Beispiel 26 Stufe 3 beschriebenen Bedingungen umgesetzt. Verbindung (56) wurde in einer Ausbeute von 76 % der Theorie erhalten.

Schmelzpunkt: 127° C - 128° C

5. Stufe:

(1RS, 2RS, 4SR)-4-(Cyclopentyl-ethyl)-2-dimethylaminomethyl-1-(3-methoxyphenyl)-cyclohexanol, Hydrochlorid (52)

Die Base der Verbindung (56) wurde mit 1-Brom-3-methoxy-benzol entsprechend den in Beispiel 1 beschriebenen Bedingungen umgesetzt. Die erhaltene Base wurde über eine Kieselgelsäule mit Diisopropylether gereinigt und in Ether aufgenommen. Nach Zugabe Trimethylchlorsilan/Wasser wurde Verbindung (52) in einer Ausbeute von 54 % der Theorie erhalten.

Schmelzpunkt: 154° C - 156° C

**Beispiel 30:**

(1RS, 2RS, 4SR)-3-[4-(2-Cyclopentyl-ethyl)-2-dimethylaminomethyl-1-hydroxy-cyclohexyl]-phenol, Hydrochlorid (57)

Die Reaktion wurde in einer Stickstoffatmosphäre unter Feuchtigkeitsausschluß durchgeführt. 1,44 g (4 mmol) der Base der Verbindung (52) wurden in 15 ml Toluol gelöst. Es wurden unter Rühren 25 ml 20 %iges Diisobutylaluminiumhydrid in Toluol (35 mmol) zugetropft. Anschließend wurde 6,5 Stunden unter Rückfluß gekocht und abgekühlt. Bei einer Temperatur zwischen 0° C und 10° C wurden 5 ml Ethanol, anschließend 5 ml Ethanol/Wasser 1:1 und 35 ml Toluol zugetropft. Nach einstündigem Rühren wurden die erhaltenen Salze abgesaugt und das Lösungsmittel abdestilliert. Das erhaltene Öl wurde in Essigester aufgenommen. Nach Zugabe von Trimethylchlorsilan/Wasser wurde Verbindung (57) in einer Ausbeute von 0,35 g (23 % der Theorie) erhalten.

Schmelzpunkt: 226° C - 228° C

**Beispiel 31:**

(1RS, 2RS, 4SR)-4-Cyclopentylethyl-2-dimethylaminomethyl-1-(3-ethoxyphenyl)-cyclohexanol, Hydrochlorid (58)

Die Base der Verbindung (56) wurde mit 1-Brom-3-ethoxybenzol entsprechend den in Beispiel 1 beschriebenen Bedingungen umgesetzt. Die erhaltene Base wurde über eine Kieselgelsäule mit Diisopropylether gereinigt und in 2-Butanon/Ether aufgenommen. Nach Zugabe von Trimethylchlorsilan/Wasser Verbindung (58) in einer Ausbeute von 52 % der Theorie erhalten.

Schmelzpunkt: 152° C - 152,5° C

**Beispiel 32:**

(1RS, 2RS, 4SR)-4-(2-Cyclopentyl-ethyl-1-(3-cyclopentyloxy-phenyl)-2-dimethylaminomethyl-cyclohexanol, Hydrochlorid (59)

Die Base der Verbindung (56) wurde mit 1-Brom-3-cyclopentyloxy-benzol entsprechend den in Beispiel 1 beschriebenen Bedingungen umgesetzt. Die erhaltene Base wurde über eine Kieselgelsäule mit Dilsopropylether gereinigt und in Essigester/Diisopropylether aufgenommen. Nach Zugabe von Trimethylchlorsilan/Wasser wurde Verbindung (59) in einer Ausbeute von 67 % der Theorie erhalten.

Schmelzpunkt: 140° C - 143° C

**Beispiel 33:**

(1RS, 2RS, 4SR)-4-Cyclopentylmethoxy-1-(3-cyclopentyloxy-phenyl)-2-dimethylaminomethyl-cyclohexanol, Hydrochlorid (60)

1. Stufe:

4-(Cyclopentylmethyloxy)-cyclohexanon (61)

23,7 g (150 mmol) 1,4-Dioxa-spiro[4.5]decan-8-ol wurden in 120 ml Dimethylformamid gelöst und in Stickstoffatmosphäre 1 Stunde bei Raumtemperatur mit 7,2 g (159 mmol) 50 %igem Natriumhydrid in Mineralöl gerührt. Anschließend wurden 38 g (150 mmol) Toluol-4-sulfonsäure-cyclopentylmethylester zugegeben (Krapcho, Johnson, J. Org. Chem., 36, 146,(1971)). Es wurde bei Raumtemperatur gerührt und auf eine Temperatur zwischen 5° C und 10° C abgekühlt. Anschließend wurden 125 ml Wasser zugetropft. Es wurde mit Ether extrahiert, über Natriumsulfat getrocknet und das Lösungsmittel destillativ entfernt. Das erhaltene Produkt (32 g) wurde in Diisopropylether gelöst und 20 Stunden mit 65 ml Wasser und 95 ml konzentrierter Salzsäure gerührt. Anschließend wurde mit Natriumhydrogencarbonat neutralisiert, mit Ether extrahiert und über Natriumsulfat getrocknet. Das erhaltene Öl (23,4 g) wurde über eine Kieselgelsäule mit Diisopropylether/ n-Hexan 1:1 gereinigt. Es wurden 17,5 g Verbindung (61) 60 % der Theorie erhalten.

2. Stufe:

4-(Cyclopentylmethyloxy)-2-dimethylaminomethyl-cyclohexanon (62)

Die Verbindung (61) wurde gemäß den in Beispiel 26 Stufe 3 beschriebenen Bedingungen umgesetzt. Verbindung (62) wurde in einer Ausbeute von 90 % der Theorie erhalten.

3. Stufe:

(1RS, 2RS, 4SR)-4-Cyclopentylmethoxy-1-(3-cyclopentyloxy-phenyl)-2-dimethylaminomethyl-cyclohexanol, Hydrochlorid (60)

Verbindung (62) wurde mit 1-Brom-3-cyclopentyloxy-benzol entsprechend den in Beispiel 1 beschriebenen Bedingungen umgesetzt. Es entstand ein Gemisch aus cis- und trans-Isomeren, das über eine Kieselgelsäule mit Ether/Methanol 20:1 getrennt und gereinigt wurde. Anschließend wurde das cis-Isomere in 2-Butanon aufgenommen und Trimethylchlorsilan/Wasser zugegeben. Nach Umkristallisieren aus 2-Butanon wurde Verbindung 60 in einer Ausbeute von 12 % der Theorie erhalten.

Schmelzpunkt: 181° C - 182,5° C

**Beispiel 34:**

(E)-(1RS, 2RS)-4-Benzyliden-2-dimethylaminomethyl-1-(3-methoxy-phenyl)-cyclohexanol, Hydrochlorid [E(63)] und

(Z)-(1RS, 2RS)-4-Benzyliden-2-dimethylaminomethyl-1-(3-methoxy-phenyl)-cyclohexanol, Hydrochlorid [Z(63)]

1. Stufe:

7-Dimethylaminomethyl-1,4-dioxa-spiro[4.5]decan-8-on, Hydrochlorid (64)

130 g (0,83 mol) 1,4-Dioxa-spiro[4.5]-decan-8-on und 79,5 g ( 0,83 mol) Dimethylammoniummethylenchlorid wurden in 500 ml Acetonitril bei Raumtemperatur gerührt. Nach Zugabe von 1 ml Acetylchlorid wurde 3 Stunden bei Raumtemperatur gerührt, wobei eine farblose, klare Lösung entstand. Anschließend wurde 1 l Ether zum Reaktionsgemisch getropft. Es wurden 203 g (98 % der Theorie) 7-Dimethylaminomethyl-1,4-dioxa-spiro[4.5]decan-8-on-Hydrochlorid (64) in kristalliner Form erhalten.

2. Stufe:

7-Dimethyalaminomethyl-8-(3-methoxy-phenyl)-1,4-dioxa-spiro[4.5]decan-8-ol (65)

Zu 16,4 g (0,68 mol) Magnesiumspänen in 50 ml Tetrahydrofuran wurden tropfenweise 85 ml (0,68 mol) 1-Brom-3-methoxy-benzol, gelöst in 350 ml Tetrahydrofuran, gegeben. Es wurde eine Stunde unter Rückfluß gekocht und auf eine Temperatur zwischen 5° C und 10 °C abgekühlt. Aus 7-Dimethylaminomethyl-1,4-dioxa-spiro[4.5]decan-8-on, Hydrochlorid (64) wurde mit Dichlormethan/Natronlauge die Base freigesetzt und nach dem Trocknen der Lösung Dichlormethan destillativ entfernt. 95 g (0,45 mol) der erhaltenen Base wurden in 150 ml Tetrahydrofuran gelöst und zur Grignard-Lösung gegeben. Es wurde über Nacht stehen gelassen und anschließend auf eine Temperatur zwischen 5° C und 10 °C abgekühlt. Durch Zugabe von 600 ml 20 %iger Ammoniumchloridlösung wurde die Grignard-Lösung zersetzt. Es wurde mit 500 ml Tetrahydrofuran verdünnt, die organische Phase abgetrennt, die wäßrige Phase zweimal mit Ether extrahiert. Nach Trocknung über Natriumsulfat und destillativer Entfernung des Lösungsmittels wurde der Rückstand (156 g) auf eine Kieselgelsäule gegeben und mit n-Hexan/Diisopropylether 4:1 und anschließend mit Ethlacetat/Methanol 1:1 eluiert. Es wurden 137 g (94 % der Theorie) Base (65) als hellgelbes, viskoses Öl erhalten.

3. Stufe:

3-Dimethylaminomethyl-4-hydroxy-4-(3-methoxy-phenyl)-cyclohexanon (66)

78 g (0,24 mol) 7-Dimethyalaminomethyl-8-(3-methoxyphenyl)-1,4-dioxa-spiro[4.5]decan-8-ol (65) wurden in 500 ml Tetrahydrofuran gelöst und auf eine Temperatur zwischen 0° C und 5° C gekühlt. Innerhalb von 30 Minuten wurden 200 ml wäßrige Salzsäure (konz. Salzsäure/Wasser 2:1) zugegeben. Es wurde 12 Stunden bei Raumtemperatur gerührt und anschließend auf eine Temperatur zwischen 0° C und 5° C abgekühlt. Nach Zugabe von 250 ml konzentrierter Natronlauge wurde dreimal mit Ether extrahiert und über Natriumsulfat getrocknet. Nach destillativer Entfernung des Lösungsmittels wurde der Rückstand (66 g) auf eine Kieselgelsäule gegeben und nacheinander mit Diisopropylether, Diisopropylether/Ether 1:1 und Ethylacetat/Methanol 1:1 eluiert. Es wurden 36 g Base (66)(48 % der Theorie) als hellgelbes, viskoses Öl erhalten.

4. Stufe:

(E)-(1RS, 2RS)-4-Benzyliden-2-dimethylaminomethyl-1-(3-methoxy-phenyl)-cyclohexanol, Hydrochlorid [E(63)] und

(Z)-(1RS, 2RS)-4-Benzyliden-2-dimethylaminomethyl-1-(3-methoxy-phenyl)-cyclohexanol, Hydrochlorid [Z(63)]

16,45 g (72,1 mmol) Benzyl-phosphonsäurediethylester wurden in 70 ml Dimethylformamid gelöst und unter Eisbadkühlung mit 4,28 g (80 mmol) Natriummethanolat versetzt. Nach 30 minütigem Rühren wurden 10 g (36 mmol) 3-Dimethylaminomethyl-4-hydroxy-4-(3-methoxy-phenyl)-cyclohexanon (66), gelöst in 20 ml Dimethylformamid, unter Eisbadkühlung, zugetropft. Anschließend wurde auf Raumtemperatur erwärmt und 24 Stunden bei dieser Temperatur gerührt. Zur Zersetzung wurde unter Eisbadkühlung ein Gemisch aus 45 ml Wasser und 25 ml Methanol zugetropft. Nach dreimaliger Extraktion mit Ether wurde mit Wasser gewaschen und über Magnesiumsulfat getrocknet. Anschließend wurde der Ether destillativ entfernt und der Rückstand (15,8 g) auf eine Kieselgelsäule gegeben. Die Elution mit Diisopropylether/Methanol 7:1 ergab 4,9 g [Z(63)]- und 3,8 g [E(63)]-Verbindung, aus denen mit Trimethylchlorsilan/Wasser in 2-Butanon die Hydrochloride erhalten wurden.

| | |
|---|---|
| [Z(63)]: | Ausbeute: 5,0 g (35 % der Theorie)<br>Schmp.: 191° C - 192 °C |
| [E(63)]: | Ausbeute: 3,9 g (28 % der Theorie),<br>Schmp.: 220° C - 221 °C |

**Beispiel 35:**

(1RS, 2RS, 4SR)-4-(3,4-Dichloro-benzyloxy)-2-dimethylamino-methyl-1-(3-methoxy-phenyl)-cyclohexanol, Hydrochlorid (67)

1. Stufe:

2-Dimethylaminomethyl-1-(3-methoxy-phenyl)-cyclohexan-1,4-diol, Hydrochlorid (68)

10 g (36 mmol) Verbindung (66) wurden in 80 ml Isopropanol gelöst und auf 10° C gekühlt. Unter Rühren wurden 0,56 g (15 mmol) Natriumborhydrid zugegeben. Anschließend wurde bei Raumtemperatur zwei Stunden gerührt. Unter Eisbadkühlung wurden 20 ml verdünnter Salzsäure (konz. $HCl:H_2O$ 1:4) und anschließend 10 ml 20 %ige Natronlauge zugegeben. Es wurde zweimal mit Dichlormethan extrahiert. Nach Trocknung und destillativer Entfernung des Lösungsmittels wurde das erhaltene Rohprodukt (10,3 g) in 2-Butanon gelöst und zur Diastereomerentrennung mit Trimethylchlorsilan/Wasser in das Hydrochlorid überführt. Es kristallisierten 10,2 g (90 % der Theorie) der Verbindung (68) aus.

2. Stufe:

(1RS, 2RS, 4SR)-4-(3,4-Dichloro-benzyloxy)-2-dimethylamino-methyl-1-(3-methoxy-phenyl)-cyclohexanol, Hydrochlorid (67)

Aus Verbindung (68) wurde mit Dichlormethan/Natronlauge die Base freigesetzt und nach dem Trocknen der Lösung das Dichlormethan destillativ entfernt. 2,8 g (10 mmol) der erhaltenen Base wurden in 10 ml Dimethylformamid gelöst und mit 480 mg Natriumhydrid (50 %ig) versetzt. Anschließend wurde zwei Stunden bei 55 °C gerührt. Es wurden 1,38 ml (1,95 g, 10 mmol) 1,2-Dichlor-4-chlormethyl-benzol zugetropft. Nach zwei Stunden Rühren bei 55° C wurde auf Raumtemperatur abgekühlt, auf Eis/Wasser gegeben, dreimal mit Ether extrahiert, mit Natronlauge gewaschen und anschließend mit Wasser gewaschen und über Natriumsulfat getrocknet. Nach destillativer Entfernung des Lösungsmittels wurden 4,0 g Rohprodukt erhalten, das auf eine Kieselgelsäule gegeben wurde. Die Elution mit Diisopropylether/Methanol 7:1 ergab 3,0 g Base, aus der mit Trimethylchlorsilan/Wasser in 2-Butanon die Verbindung mit der Formel (67) (3,1 g, 65 % der Theorie) mit einem Schmelzpunkt von 196 °C - 197° C erhalten wurde.

**Beispiele 36 - 61:**

In der nachfolgenden Tabelle sind die Beispiele 36 - 61 zusammengefaßt. Die aufgeführten Verbindungen wurden aus den entsprechenden Ausgangsverbindungen unter den in den Beispielen 1 - 35 beschriebenen Bedingungen hergestellt.

| Bsp. | Verbindung | X | $R^1$ | Y | $R^2$ | Schmelzpunkt | $[\alpha]_D^{RT}$ | hergestellt analog Bsp. |
|---|---|---|---|---|---|---|---|---|
| 36 | (1RS, 2RS, 4SR)-3-(4-Cyclopentylmethoxy)-2-dimethylaminomethyl-(1-hydroxy-cyclohexyl)-phenol, Hydrochlorid (69) | O | H | O | Cyclopentylmethyl | 188 - 191°C | - | 6 |
| 37 | (-)-(1S, 2S, 4R)-4-(2-Cyclopentylethyl)-1-(3-cyclopentyloxyphenyl)-2-dimethylaminomethylcyclohexanol, Hydrochlorid (70) | O | Cyclopentyl | $CH_2$ | Cyclopentylmethyl | 165,5 - 167°C | -21° | 32+2 |
| 38 | (+)-(1R, 2R, 4S)-4-(2-Cyclopentylethyl)-1-(3-cyclopentyloxyphenyl)-2-dimethylaminomethylcyclohexanol, Hydrochlorid (71) | O | Cyclopentyl | $CH_2$ | Cyclopentylmethyl | 193 - 194°C | +22° | 32+2 |
| 39 | (-)-(1S, 2S, 4R)-4-(Cyclopentylethyl)-2-dimethylaminomethyl-1-(3-methoxyphenyl)-cyclohexanol, Hydrochlorid (72) | O | Methyl | CH2 | Cyclopentylmethyl | 212 - 212,5°C | -24° | 29+2 |

| Bsp. | Verbindung | X | $R^1$ | Y | $R^2$ | Schmelz-punkt | $[\alpha]_D^{RT}$ | hergestellt analog Bsp. |
|---|---|---|---|---|---|---|---|---|
| 40 | (+)-(1R, 2R, 4S)-4-(Cyclopentylethyl)-2-dimethylaminomethyl-1-(3-methoxyphenyl)-cyclohexanol, Hydrochlorid (73) | O | Methyl | $CH_2$ | Cyclopentylmethyl | 211,5 - 212,5°C | +27° | 29+2 |
| 41 | (-)-(1S, 2S, 4R)-4-(Cyclopentylethyl)-2-dimethylaminomethyl-1-(3-ethoxyphenyl)-cyclohexanol, Hydrochlorid (74) | O | Ethyl | $CH_2$ | Cyclopentylmethyl | 191 - 191,5°C | -21° | 31+2 |
| 42 | (+)-(1R, 2R, 4S)-4-(Cyclopentylethyl)-2-dimethylaminomethyl-1-(3-ethoxyphenyl)-cyclohexanol, Hydrochlorid (75) | O | Ethyl | $CH_2$ | Cyclopentylmethyl | 191°C | +26° | 31+2 |
| 43 | (-)-(1S, 2S, 4R)-4-Benzyloxy-2-dimethylaminomethyl-1-[3-(2-fluoro-ethoxy)-phenyl]-cyclohexanol, Hydrochlorid (76) | O | 2-Fluorethyl | O | Benzyl | 161 - 163°C | -17° | 3+2 |

| Bsp. | Verbindung | X | $R^1$ | Y | $R^2$ | Schmelz-punkt | $[\alpha]_D^{RT}$ | hergestellt analog Bsp. |
|---|---|---|---|---|---|---|---|---|
| 44 | (+)-(1R, 2R, 4S)-4-Benzyloxy-2-dimethylaminomethyl-1-[3-(2-fluoro-ethoxy)-phenyl]-cyclohexanol, Hydrochlorid (77) | O | 2-Fluor-ethyl | O | Benzyl | 162 - 164°C | +17° | 3+2 |
| 45 | (1RS, 2RS, 4SR)-2-Dimethylaminomethyl-1-(3-methoxyphenyl)-4-propoxycyclohexanol, Hydrochlorid (76) | O | Methyl | O | n-Propyl | 148° - 150°C | - | 10 |
| 46 | (1RS, 2RS, 4SR)-4-(4-Chloro-benzyloxy)-2-dimethylamino-methyl-1-(3-methoxyphenyl)-cyclohexanol, Hydrochlorid (79) | O | Methyl | O | 4-Chlor-benzyl | 156°C | - | 1 |
| 47 | (1RS, 2RS, 4SR)-2-Dimethylaminomethyl-4-(4-fluoro-benzyl-oxy)-1-(3-methoxyphenyl)-cyclohexanol, Hydrochlorid (80) | O | Methyl | O | 4-Fluor-benzyl | 167°C | - | 1 |

| Bsp. | Verbindung | X | $R^1$ | Y | $R^2$ | Schmelz-punkt | $[\alpha]_D^{RT}$ | herge-stellt analog Bsp. |
|---|---|---|---|---|---|---|---|---|
| 48 | (1RS, 2RS, 4SR)-2-Dimethylaminomethyl-4-methoxy-1-(3-methoxyphenyl)-cyclohexanol, Hydrochlorid (81) | O | Methyl | O | Methyl | 188°C | - | 10 |
| 49 | (1RS, 2RS, 4SR)-4-(4-tert-Butyl-benzyloxy)-2-dimethylaminomethyl-1-(3-methoxyphenyl)-cyclohexanol, Hydrochlorid (82) | O | $CH_3$ | O | 4-tert-Butyl-benzyl | 189 - 190°C | - | 1 |
| 50 | (+)-(1R, 2R, 4S)-4-Benzyloxy-2-dimethylaminomethyl-1-(3-isopropoxyphenyl)-cyclohexanol, Hydrochlorid (83) | O | Iso-propyl | O | Benzyl | 167,5 - 170°C | +20° | 4+2 |
| 51 | (-)-(1S, 2S, 4R)-4-Benzyloxy-2-dimethylaminomethyl-1-(3-isopropoxyphenyl)-cyclohexanol, Hydrochlorid (84) | O | Iso-propyl | O | Benzyl | 167 - 171°C | -19,1° | 4+2 |

| Bsp. | Verbindung | X | $R^1$ | Y | $R^2$ | Schmelz-punkt | $[\alpha]_D^{RT}$ | hergestellt analog Bsp. |
|---|---|---|---|---|---|---|---|---|
| 52 | (+)-(1R, 2R, 4S)-3-(4-Benzyl-oxy-2-dimethylaminomethyl-1-hydroxycyclohexyl)-phenol, Hydrochlorid (85) | O | H | O | Benzyl | 199 - 202°C | +21,2° | 6+2 |
| 53 | (-)-(1S, 2S, 4R)-3-(4-Benzyl-oxy-2-dimethylaminomethyl-1-hydroxycyclohexyl)-phenol, Hydrochlorid (86) | O | H | O | Benzyl | 200 - 203°C | -16,1° | 6+2 |
| 54 | (+)-(1R, 2R, 4S)-4-Benzyloxy-1-(3-cyclopentyloxyphenyl)-2-dimethylaminomethyl-cyclo-hexanol, Hydrochlorid (87) | O | Cyclo-pentyl | O | Benzyl | 115 - 129°C | +18,6° | 32+2 |
| 55 | (-)-(1S, 2S, 4R)-4-Benzyloxy-1-(3-cyclopentyloxyphenyl)-2-dimethylaminomethyl-cyclo-hexanol, Hydrochlorid (88) | O | Cyclo-pentyl | O | Benzyl | 128 - 142°C | -18,4° | 32+2 |

| Bsp. | Verbindung | X | $R^1$ | Y | $R^2$ | Schmelz-punkt | $[\alpha]_D^{RT}$ | hergestellt analog Bsp. |
|---|---|---|---|---|---|---|---|---|
| 56 | (1RS, 2RS, 4SR)-3-[4-(4-Chloro-benzyloxy)-2-dimethylamino-methyl-1-hydroxycyclohexyl]-phenol, Hydrochlorid (89) | O | H | O | 4-Chlor-benzyl | 242 - 246°C | - | 6 |
| 57 | (-)-(1S, 2S, 4R)-2-Dimethylaminomethyl-4-(4-fluoro-benzyl-oxy)-1-(3-methoxyphenyl)-cyclo-hexanol, Hydrochlorid (90) | O | Methyl | O | 4-Fluor-benzyl | 232 - 234°C | -20,5° | 47+2 |
| 58 | (+)-(1R, 2R, 4S)-2-Dimethyl-aminomethyl-4-(4-fluoro-benzyl-oxy)-1-(3-methoxyphenyl)-cyclohexanol, Hydrochlorid (91) | O | Methyl | O | 4-Fluor-benzyl | 232,5 - 234°C | +20,3° | 47+2 |
| 59 | (-)-(1S, 2S, 4R)-4-(4-Chloro-benzyloxy)-2-dimethylamino-methyl-1-(3-methoxyphenyl)-cyclohexanol, Hydrochlorid (92) | O | Methyl | O | 4-Chlor-benzyl | 196,5 - 198°C | -19,2° | 46+2 |

| Bsp. | Verbindung | X | $R^1$ | Y | $R^2$ | Schmelzpunkt | $[\alpha]_D^{RT}$ | hergestellt analog Bsp. |
|---|---|---|---|---|---|---|---|---|
| 60 | (+)-(1R, 2R, 4S)-4-(4-Chlorobenzyloxy)-2-dimethylaminomethyl-1-(3-methoxyphenyl)-cyclohexanol, Hydrochlorid (93) | O | Methyl | O | 4-Chlorbenzyl | 196,5 - 197,5°C | +20,7° | 46+2 |
| 61 | (1RS, 2RS, 4SR)-4-(4-Chlorobenzyloxy)-2-dimethylaminomethyl-1-(3-isopropoxyphenyl)-cyclohexanol, Hydrochlorid (94) | O | Isopropyl | O | 4-Chlorbenzyl | 127 - 129°C | - | 4 |

**Pharmakologische Untersuchungen**

**Analgesieprüfung im Tail-flick-Test an der Maus**

Die analgetische Wirksamkeit der erfindungsgemäßen Verbindungen wurde im Wärmestrahl (Tail-flick)-Test an der Maus nach der Methode von D'Amour und Smith (J. Pharm. Exp. Ther. 72, 74 - 79 (1941) ermittelt. Dazu wurden männliche NMRI-Mäuse mit einem Gewicht zwischen 20 und 24 g verwendet. Die Tiere wurden einzeln in spezielle Testkäfige gesetzt und die Schwanzbasis einem focussierten Wärmestrahl einer elektrischen Lampe (Rhema Analgesiemeter Typ 3010) ausgesetzt. Die Lampenintensität wurde so eingestellt,, daß die Zeit vom Einschalten der Lampe bis zum plötzlichen Wegzucken des Schwanzes (Schmerzlatenz) bei unbehandelten Tieren 3 - 5 Sekunden betrug. Vor Gabe einer erfindungsgemäßen Verbindung wurden die Tiere innerhalb von fünf Minuten zweimal vorgetestet und der Mittelwert dieser Messungen als Vortestmittelwert berechnet. Die Schmerzmessung wurde 20, 40 und 60 Minuten nach intravenöser Gabe durchgeführt. Bei Anstieg der Schmerzlatenz wurde die maximale Expositionszeit auf 12 Sekunden beschränkt und die Zunahme der Latenzzeit auf ≥150 % des Vortestmittelwertes als analgetische Wirkung gewertet. Zur Bestimmung der Dosisabhängigkeit wurde die jeweilige erfindungsgemäße Verbindung in 3 - 5 logarithmisch ansteigenden Dosen, die jeweils die Schwellen- und die maximale Wirkdosis einschlossen, appliziert und aus der Anzahl der analgetischen Tiere nach der Methode von Litchfield und Wilcoxon (J. Pharm. Exp. Ther. 96, 99 - 1123 (1949)) die $ED_{50}$-Werte bestimmt. Die $ED_{50}$-Ermittlung erfolgte im Wirkmaximum 20 Minuten nach intravenöser Substanzgabe.

Alle eingesetzten erfindungsgemäßen Verbindungen zeigten eine ausgeprägte analgetische Wirkung. Die Ergebnisse sind in der nachfolgenden Tabelle zusammengefaßt.

Tabelle

| Analgesieprüfung im Tail-flick-Test an der Maus | | |
|---|---|---|
| Beispiel | erfindungsgemäße Verbindung | $ED_{50}$ (mg/kg intravenös) |
| 2 | [(+)1] | 0,049 |
| 2 | [(-)1] | 0,822 |
| 8 | [(+)6] | 0,190 |
| 11 | (14) | 0,379 |
| 22 | (35) | 2,430 |
| 33 | (60) | 2,460 |
| 38 | (71) | 3,350 |
| 52 | (85) | 0,068 |
| 60 | (93) | 0,370 |
| Tramadol | - | 14,700 |

**Patentansprüche**

1. 1-Phenyl-2-dimethylaminomethyl-cyclohexan-1-olverbindungen der Formel I

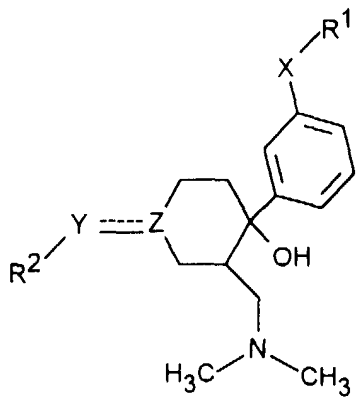

in der

X O oder S bedeutet,
$R^1$ H, $C_{1-6}$-Alkyl, $C_{2-6}$-Alkenyl, $C_{5-7}$-Cycloalkyl oder halogeniertes $C_{1-6}$-Alkyl bedeutet, die Gruppierung

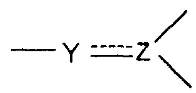

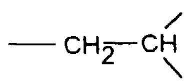 , 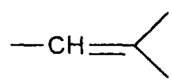 oder 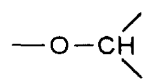

bedeutet,
$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,

in Form ihrer Basen oder Salze von physiologisch verträglichen Säuren.

**2.** 1-Phenyl-2-dimethylaminomethyl-cyclohexan-1-olverbindungen der Formel I gemäß Anspruch 1, dadurch gekennzeichnet, daß
$R^1$ H, $C_{1-4}$-Alkyl, 2'-Methyl-2'-propenyl, Cyclopentyl oder Fluorethyl bedeutet mit der Maßgabe, daß $R^1$ $C_{1-4}$-Alkyl ist, wenn X S bedeutet,
$R^2$ $C_{1-4}$-Alkyl, $C_{2-4}$-Alkenyl, Cyclopentylmethyl, Phenyl, $C_{1-4}$-Alkoxyphenyl, Benzyl, $C_{1-4}$-Alkylbenzyl, einfach oder doppelt halogeniertes Phenyl oder einfach oder doppelt halogeniertes Benzyl bedeutet.

**3.** 1-Phenyl-2-dimethylaminomethyl-cyclohexan-1-olverbindungen der Formel I gemäß Anspruch 1 oder 2, dadurch gekennzeichnet, daß
$R^1$ H, Methyl, Ethyl, Isopropyl, 2'-Methyl-2'-propenyl, Cyclopentyl oder Fluorethyl bedeutet, mit der Maßgabe, daß $R^1$ Methyl ist, wenn X S bedeutet,
$R^2$ Methyl, Propyl, 2'-Methyl-propyl, Allyl, 2'-Methyl-2'-propenyl, Cyclopentylmethyl, Phenyl, 3-Methoxyphenyl, Benzyl, 4-tert-Butylbenzyl, 4-Chlorbenzyl, 4-Fluorbenzyl oder 3,4-Dichlorbenzyl bedeutet.

**4.** 1-Phenyl-2-dimethylaminomethyl-cyclohexan-1-olverbindungen der Formel I gemäß einem der Ansprüche 1 bis 3, dadurch gekennzeichnet, daß
$R^1$ H, Methyl oder Cyclopentyl bedeutet, mit der Maßgabe, daß $R^1$ Methyl ist, wenn X S bedeutet,
die Gruppierung

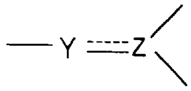

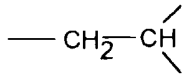 oder 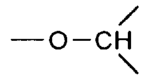

bedeutet und

$R^2$ Cyclopentylmethyl, Benzyl und 4-Chlorobenzyl darstellt.

**5.** 1-Phenyl-2-dimethylaminomethyl-cyclohexan-1-olverbindungen der Formel I gemäß Anspruch 4, dadurch gekennzeichnet, daß X O bedeutet, $R^1$ H oder Methyl bedeutet,
die Gruppierung

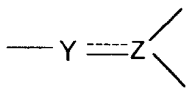

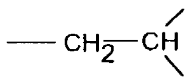 oder 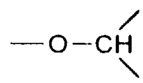

bedeutet und

$R^2$ Benzyl ist.

**6.** 1-Phenyl-2-dimethylaminomethyl-cyclohexan-1-olverbindungen nach einem der Ansprüche 1 bis 5, dadurch gekennzeichnet, daß die Verbindungen die Konfiguration der Formel Ia

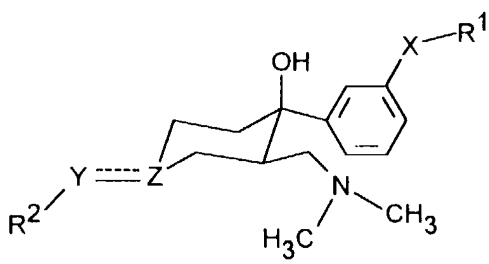

haben.

**7.** Arzneimittel enthaltend als Wirkstoff wenigstens eine Verbindung gemäß den Ansprüchen 1 bis 6 und gegebenenfalls weitere Wirkstoffe.

**8.** Arzneimittel nach Anspruch 7 mit analgetischer Wirkung.

**9.** Verfahren zur Herstellung einer 1-Phenyl-2-dimethylaminomethyl-cyclohexan-1-olverbindung der Formel I

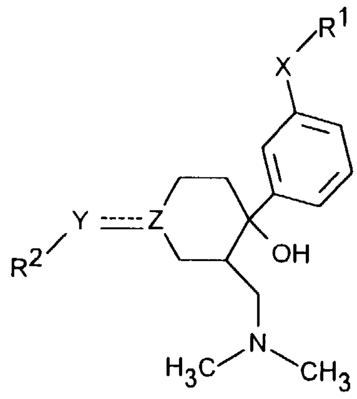

in der

X O oder S bedeutet,

$R^1$ $C_{1-6}$-Alyl, $C_{2-6}$-Alkeyl, $C_{5-7}$-Cycloalkyl, halogeniertes $C_{1-6}$-Alkyl, Benzyl, Diarylalkylsilyl oder Trialkylsilyl bedeutet,
die Gruppierung

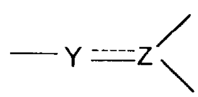 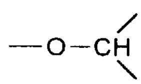

ist,

$R^2$ $C_{1-6}$ A1ky], $C_{2-6}$-Alkenyl, $C_{5-7}$-Cycloalkylmethyl, substituiertes oder unsubstituiertes Phenyl oder substituiertes oder unsubstituiertes Benzyl bedeutet,

dadurch gekennzeichnet, daß man ein 4-substituiertes Cyclohexan-1-on der Formel IIa

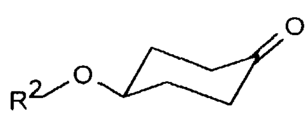

herstellt, indem man
entweder eine Verbindung der Formel IV

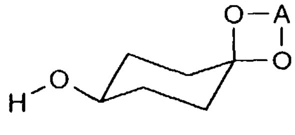

in der A ein verzweigter oder unverzweigter $C_nH_{2n}$-Rest ist und n eine ganze Zahl von 2 bis 6 bedeutet, mit einer Verbindung der Formel III

$$R^2—G$$

in der G Cl, Br, 1 oder Toluolsulfonyloxy ist, alkyliert und anschließend mittels Protonenkatalyse deacetalisiert, oder eine Verbindung der Formel V

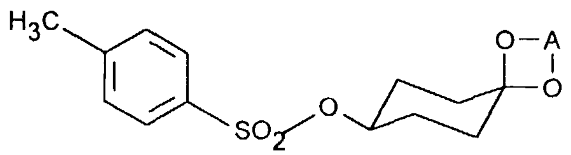

in der A ein verzweigter oder unverzweigter $C_nH_{2n}$-Rest ist und n eine ganze Zahl zwischen 2 und 6 bedeutet, mit einem Alkoholat, das man aus einem Alkohol der Formel VI

$R^2$—OH

herstellt, alkyliert und anschließend mittels Protonenkatalyse zu einer Verbindung der Formel IIa deacetalisiert, die erhaltene Verbindung der Formel IIa entweder mit Dimethylamlin in einer Mannich-Reaktion oder mit Dimethylammoniummethylenchlorid zu einer Verbindung der Formel VIIa

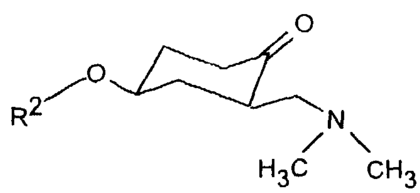

umsetzt, die man mit einer metallorganischen Verbindung der Formel VIII

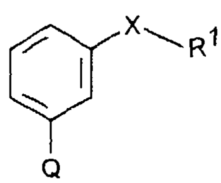

in der Q MgCl, MgBr, MgI oder Li bedeutet, in eine Verbindung der Formel I überführt.

**10.** Verfahren zur Herstellung einer 1-Phenyl-2-dimethylaminoethyl-cyclohexan-1-olverbindung der Formel I

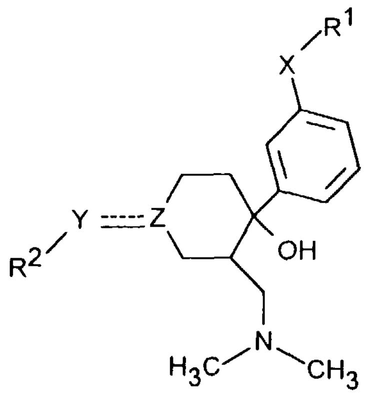

in der

X O oder S bedeutet,

$R^1$ $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet, die Gruppierung

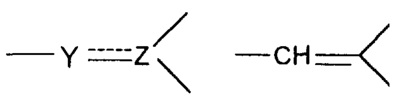

ist,

$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,

dadurch gekennzeichnet, daß man ein 4-substituiertes Cyclohexan-1-on der Formel IIb

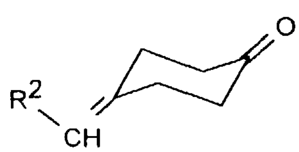

herstellt, indem man
entweder eine Grignard-Verbindung der Formel IX

$$R^2\text{-}CH_2\text{-Mg-Hal}$$

in der Hal Cl, Br oder I bedeutet, mit einem Keton der Formel X

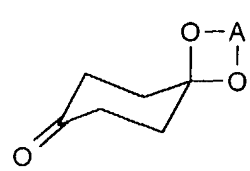

in der A eiii verzweigter oder unverzweigter $C_nH_{2n}$-Rest ist und n eine ganze Zahl zwischen 2 und 6 bedeutet zu einer Verbindung der Formel XI

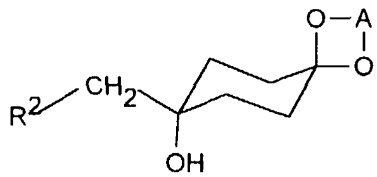

umsetzt, aus dieser anschließend mittels protononkatalysierter Deacetalisieruitg eine Keto-Verbindung der Formel XII

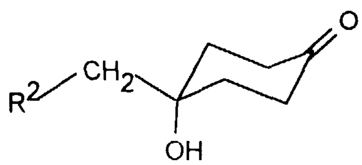

herstellt und durch anschließende Dehydratisierung die Verbindung der Formel IIb erhält oder ein Keton der Formel X mit einem Phosphoran der Formel XIII

$$R_3P{=}CH—R^2$$

in dem R Aryl bedeutet, nach Wittig zu einer Verbindung der Formel XIV

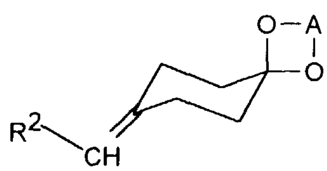

in der A ein verzweigter oder unverzweigter $C_nH_{2n}$-Rest ist und n eine ganze Zahl zwischen 2 und 6 bedeutet, umsetzt, die man anschließend mittels Protonenkatalyse in ein Keton der Formel IIb überführt,
die erhaltende Verbindung der Formel IIb entweder mit Dimethylamin in einer Mannich-Reaktion oder mit Dimethylammoniummethylenchlorid zu einer Verbindung der Formel VIIb

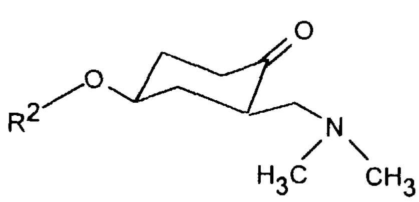

umsetzt, die man mit einer metallorganischen Verbindung der Formel VIII

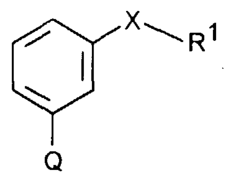

in der Q MgCl, MgBr, Mgl der Li bedeutet, in eine Verbindung der Formel I überführt.

**11.** Verfahren zur Herstellung einer 1-Phenyl-2-dimethylaminomethyl-cyclohexan-1-olverbindung der Formel I

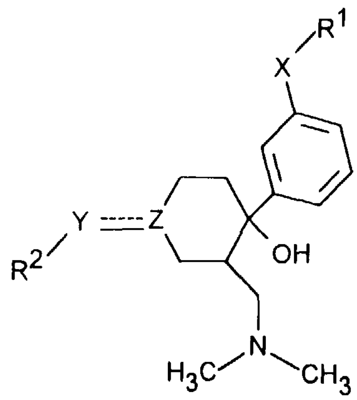

in der

X O oder S bedeutet,

$R^1$ $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet,
die Gruppierung

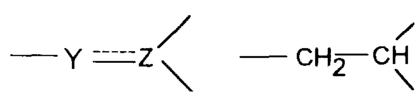

ist,

$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,

dadurch gekennzeichnet, daß man eine gemäß Anspruch 8 hergestellte Verbindung der Formel IIb

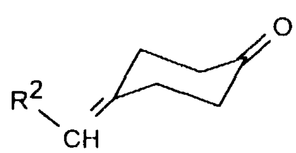

in Gegenwart eines Pd- oder Pt-Katalysators zu einem 4-substituierten Cyclohexan-1-on der Formel IIc

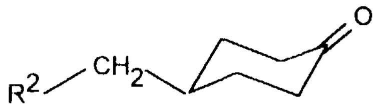

hydriert,
die erhaltene Verbindung der Formel IIc entweder mit Dimethylamin in einer Mannich-Reaktion oder mit Dimethylammoniummethylenchlorid zu einer Verbindung der Formel VIIc

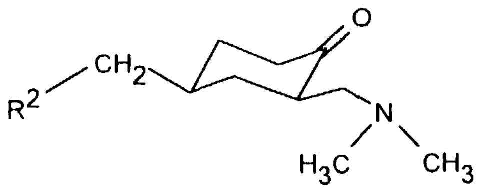

umsetzt, die man mit einer metallorganischen Verbindung der Formel VIII

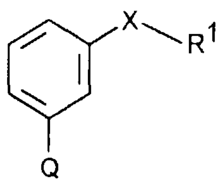

in der Q MgCl, MgBr, MgI oder Li bedeutet, in eine Verbindung der Formel I überführt.

**12.** Verfahren zur Herstellung einer 1-Phenyl-2-dimethylaminomethyl-cyclohexan-1-olverbindung der Formel I

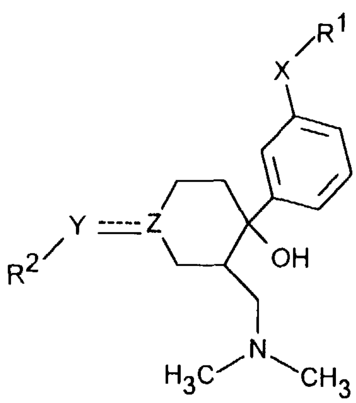

in der

X O oder S bedeutet, $R^1$ H darstellt,
die Gruppierung

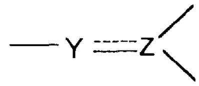

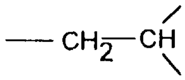 , 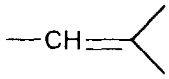 oder 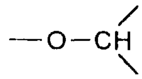

bedeutet

$R^2$ $C_{1-6}$-Alkyl, $C_{2-6}$-Alkenyl, $C_{5-7}$-Cycloalkylmethyl, substituiertes oder unsubstituiertes Phenyl oder substituiertes oder unsubstituiertes Benzyl bedeutet,

dadurch gekennzeichnet, daß man entweder
eine Verbindung der Formel I, in der $R^1$ Methyl bedeutet, mit Diisobutylaminiumhydrid umsetzt, oder eine Verbindung der Formel I, in der $R^1$ Benzyl ist, in Gegenwart eines Pt- oder Pd-Katalysators hydriert,
oder eine Verbindung der Formel I, in der $R^1$ Diarylalkylsilyl oder Trialkylsilyl bedeutet, hydrolysiert oder mit Tetra-n-butylammoniumfluorid umsetzt.

**13.** Verfahren zur Herstellung einer 1-Phenyl-2-dimethylaminomethyl-cyclohexan-1-olverbindung der Formel I

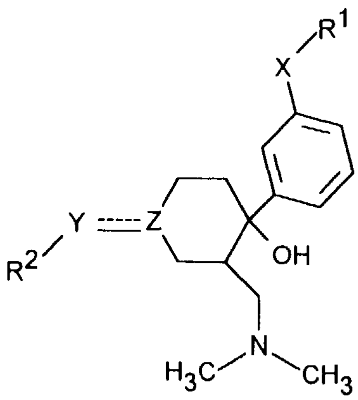

in der

X O oder S bedeutet,

$R^1$ H, $C_{1-6}$-Alkyl, $C_{2-6}$-Alkenyl, $C_{5-7}$-Cycloalkyl, halogeniertes $C_{1-6}$-Alkyl, Benzyl, Diarylalkylsilyl oder Trialkylsilyl bedeutet,
die Gruppierung

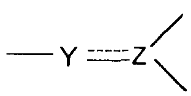

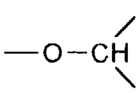 , 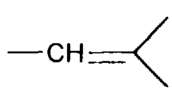 oder 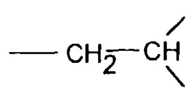

bedeutet,

$R^2$ $C_{1-6}$-Alkyl, $C_{2}$-$_{6}$-Alkenyl, $C_{5-7}$-Cycloalkylmethyl, substituiertes oder unsubstituiertes Phenyl oder substituiertes oder unsubstituiertes Benzyl bedeutet,

dadurch gekennzeichnet, daß man ein Keton der formel XV

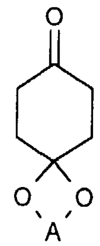

in der A ein verzweigter oder unverzweigter $C_nH_{2n}$-Rest ist und n eine ganze Zahl zwischen 2 und 6 bedeutet, mit Dimethylammoniummethylenchlorid zu einem β-Dimethylaminoketon der Formel XVI

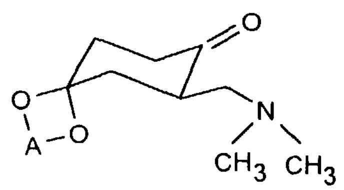

umsetzt, aus dem man anschließend mit einer metallorganischen Verbindung der Formel VIII

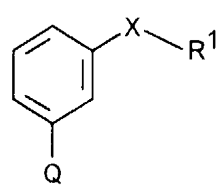

in der Q MgCl, MgBr, MgI oder Li bedeutet, eine Verbindung der Formel XVII

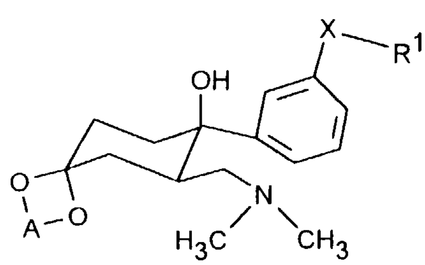

herstellt, die man anschließend mittels Protonenkatalyse zu einer Verbindung der Formel XVIII

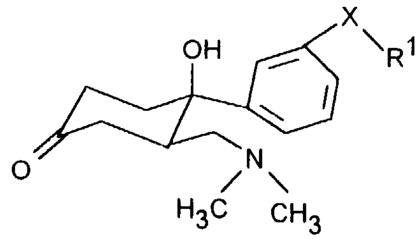

deacetalisiert,
entweder die erhaltene Verbindung der Formel XVII entweder zu einem 4-Hydroxy-Derivat der Formel XIX

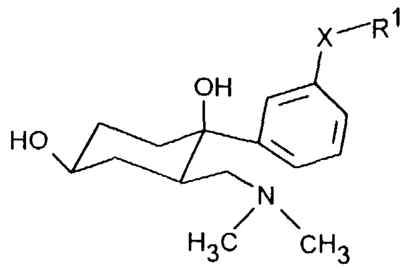

reduziert, aus dem man durch anschließende Alkoholatbildung und Umsetzung mit einer Verbindung der Formel III

$$R^2—G$$

in der G Cl, Hr, I oder Toluolsulfonyloxy ist, eine Verbindung der Formel I herstellt, in der die Gruppierung

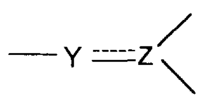 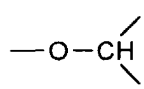

ist
oder die erhaltene Verhindung der Formel XVIII mit einer Verbindung der Formel XX

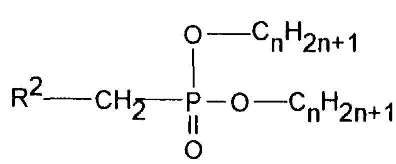

in der n eine ganze Zahl von 1 - 3 bedeutet, zu einer Verbindung der Formel I, in der die Gruppierung

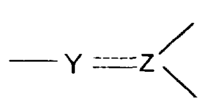 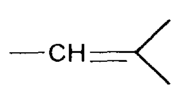

ist,
umsetzt,
die man gewünschtenfalls zu einer Verbindung der Formel I, in der
die Gruppierung

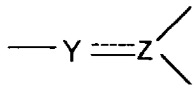 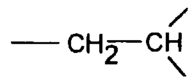

ist,
hydriert.

**14.** Verwendung einer 1-Phenyl-2-dimethylaminomethyl-cyclohexan-1-olverbindung der Formel I nach Anspruch 1 zur Herstellung eines Arzneimittels.

**15.** Verwendung nach Anspruch 14 dadurch gekennzeichnet, daß das Arzneimittel ein Schmerzmittel ist.

## Claims

**1.** 1-phenyl-2-dimethylaminomethyl-cyclohexan-1-ol compounds of formula I

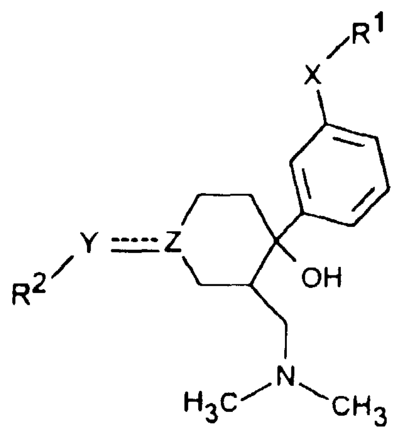

in which

X represents O or S,

$R^1$ represents H, $C_{1-6}$ alkyl, $C_{2-6}$ alkenyl, $C_{5-7}$ cycloalkyl or halogenated $C_{1-6}$ alkyl,
the

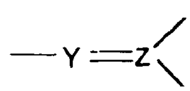

grouping represents

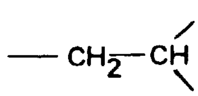 , 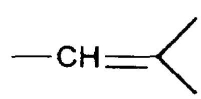 or 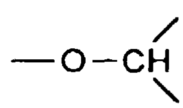

$R^2$ represents $C_{1-6}$ alkyl, $C_{2-6}$ alkenyl, $C_{5-7}$ cycloalkylmethyl, substituted or unsubstituted phenyl or substituted or unsubstituted benzyl,

in the form of their bases or salts of physiologically compatible acids.

**2.** 1-phenyl-2-dimethylaminomethyl-cyclohexan-1-ol compounds of formula I according to claim 1, characterised in that

$R^1$ represents H, $C_{1-4}$ alkyl, 2'-methyl-2'-propenyl, cyclopentyl or fluoroethyl, with the proviso that $R^1$ is $C_{1-4}$ alkyl when X represents S, and

$R^2$ represents $C_{1-4}$ alkyl, $C_{2-4}$ alkenyl, cyclopentylmethyl, phenyl, $C_{1-4}$ alkoxyphenyl, benzyl, $C_{1-4}$ alkylbenzyl, singly or doubly halogenated phenyl or singly or doubly halogenated benzyl.

**3.** 1-phenyl-2-dimethylaminomethyl-cyclohexan-1-ol compounds of formula 1 according to claim 1 or 2, characterised in that

$R^1$ represents H, methyl, ethyl, isopropyl, 2'-methyl-2'-propenyl, cyclopentyl or fluoroethyl, with the proviso that $R^1$ is methyl when X represents S, and

$R^2$ represents methyl, propyl, 2'-methyl-propyl, allyl, 2'-methyl-2'-propenyl, cyclopentylmethyl, phenyl, 3-methoxyphenyl, benzyl, 4-tert-butylbenzyl, 4-chlorobenzyl, 4-fluorobenzyl or 3,4-dichlorobenzyl.

**4.** 1-phenyl-2-dimethylaminomethyl-cyclohexan-1-ol compounds of formula I according to any one of claims 1 to 3, characterised in that

$R^1$ represents H, methyl or cyclopentyl, with the proviso that $R^1$ is methyl when X represents S,
the

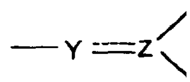

grouping represents

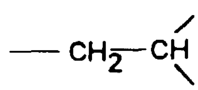

or

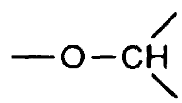

and

$R^2$ represents cyclopentylmethyl, benzyl and 4-chlorophenyl.

**5.** 1-phenyl-2-dimethylaminomethyl-cyclohexan-1-ol compounds of formula I according to claim 4, characterised in that X represents O, $R^1$ represents H or methyl,
the

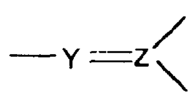

grouping represents

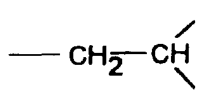

or

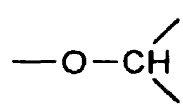

and

$R^2$ is benzyl.

**6.** 1-phenyl-2-dimethylaminomethyl-cyclohexan-1-ol compounds according to any one of claims 1 to 5, characterised in that the compounds have the configuration of formula Ia

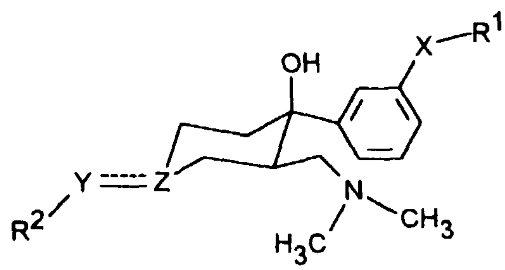

**7.** A drug containing at least one comopund according to claims 1 to 6 as an active ingredient and optionally containing further active ingredients.

**8.** A drug according to claim 7 having an analgesic effect.

**9.** A method of preparing a 1-phenyl-2-dimethylaminomethyl-cyclohexan-1-ol compound of formula I

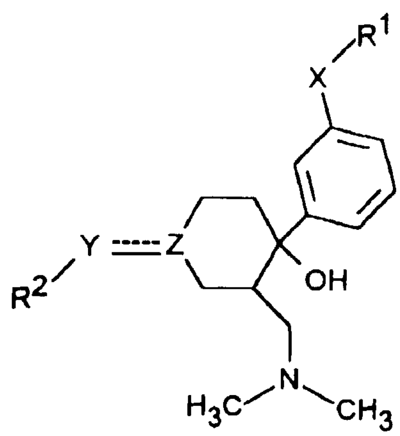

in which

X represents O or S,

$R^1$ represents $C_{1-6}$ alkyl, $C_{2-6}$ alkenyl, $C_{5-7}$ cycloalkyl, halogenated $C_{1-6}$ alkyl, benzyl, diarylalkylsilyl or tri-alkylsilyl,
the

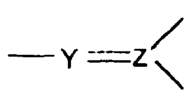

grouping is

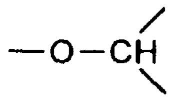 ,

and $R^2$ represents $C_{1-6}$ alkyl, $C_{2-6}$ alkenyl, $C_{5-7}$ cycloalkylmethyl, substituted or unsubstituted phenyl or substituted or unsubstituted benzyl,

characterised in that a 4-substituted cyclohexan-1-one of formula IIa

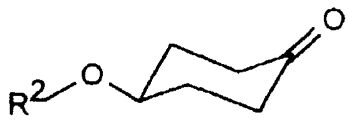

is prepared,
either by alkylating a compound of formula IV

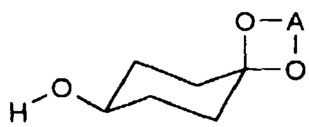 ,

in which A is a branched or unbranched $C_nH_{2n}$ radical and n represents an integer between 2 and 6, with a compound of formula III

$$R^2\text{---}G$$

in which G is Cl, Br, I or toluenesulphonyloxy, and subsequently deacetylating it by means of proton catalysis,
or by alkylating a compound of formula V

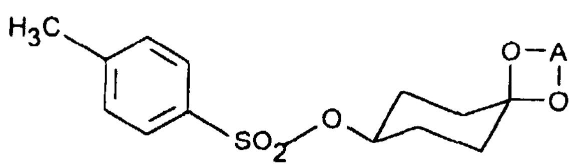 ,

in which A is a branched or unbranched $C_nH_{2n}$ radical and n represents an integer between 2 and 6, with an alcoholate which is prepared from an alcohol of formula VI

$$R^2\text{--}OH,$$

and subsequently deacetylating it by means of proton catalysis to form a compound of formula IIa,
reacting the compound of formula IIa which is obtained, either with dimethylamine in a Mannich reaction or with dimethylammonium methylene chloride, to form a compound of formula VIIa

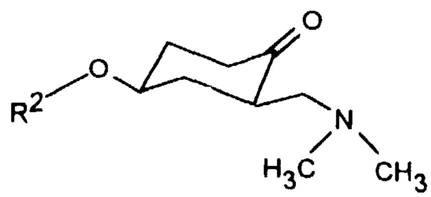

which is converted into a compound of formula I with an organometallic compound of formula VIII

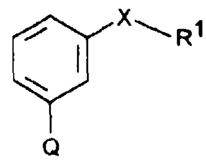

in which Q represents MgCl, MgBr, MgI or Li.

**10.** A method of preparing a 1-phenyl-2-dimethylaminomethyl-cyclohexan-1-ol compound of formula I

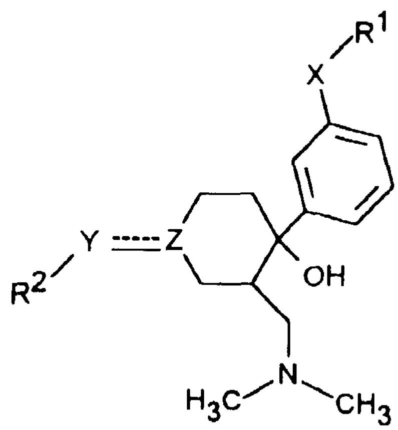

in which

X represents O or S,

$R^1$ represents $C_{1-6}$ alkyl, $C_{2-6}$ alkenyl, $C_{5-7}$ cycloalkyl, halogenated $C_{1-6}$ alkyl, benzyl, diarylalkylsilyl or trialkylsilyl,
the

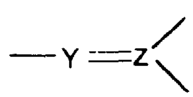

grouping is

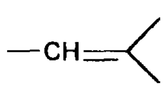 ,

and

$R^2$ represents $C_{1-6}$ alkyl, $C_{2-6}$ alkenyl, $C_{5-7}$ cycloalkylmethyl, substituted or unsubstituted phenyl or substituted or unsubstituted benzyl,

characterised in that a 4-substituted cyclohexan-1-one of formula IIb

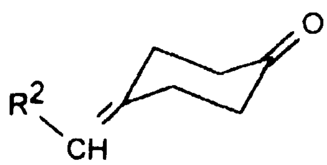

is prepared,
either by reacting a Grignard compound of formula IX

$$R^2\text{-}CH_2\text{-Mg-Hal},$$

in which Hal represents Cl, Br or I, with a ketone of formula X

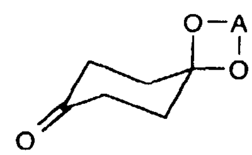

in which A is a branched or unbranched $C_nH_{2n}$ radical and n represents an integer between 2 and 6,
to form a compound of formula XI

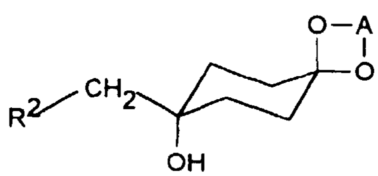

,

from which a keto compound of formula XII

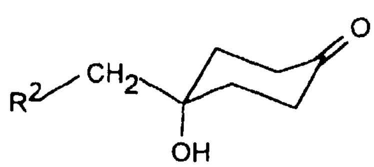

is subsequently prepared by means of proton-catalysed deacetylation, and the compound of formula IIb is obtained by subsequent dehydration,
or a ketone of formula X is subjected to a Wittig reaction with a phosphorane of formula XIII

$$R_3P = = CH\text{--}R^2,$$

in which R represents aryl, to form a compound of formula XIV

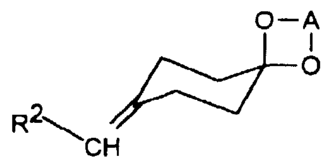

,

in which A is a branched or unbranched $C_nH_{2n}$ radical and n represents an integer between 2 and 6, which is subsequently converted by means of proton catalysis into a ketone of formula IIb,
the compound of formula IIb which is obtained is reacted, either with dimethylamine in a Mannich reaction or with dimethylammonium methylene chloride, to form a compound of formula VIIb

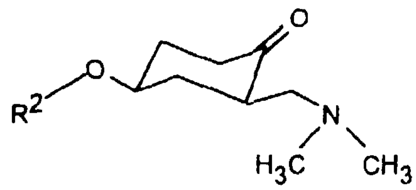

,

which is converted into a compound of formula I with an organometallic compound of formula VIII

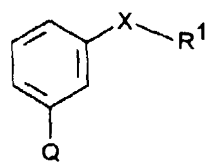

in which Q represents MgCl, MgBr, MgI or Li.

**11.** A method of preparing a 1-phenyl-2-dimethylaminomethyl-cyclohexan-1-ol compound of formula I

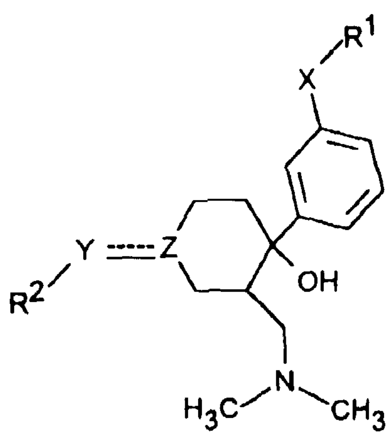

in which

X represents O or S,

$R^1$ represents $C_{1-6}$ alkyl, $C_{2-6}$ alkenyl, $C_{5-7}$ cycloalkyl, halogenated $C_{1-6}$ alkyl, benzyl, diarylalkylsilyl or trialkylsilyl,
the

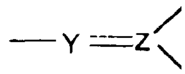

grouping is

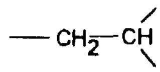
,

and

$R^2$ represents $C_{1-6}$ alkyl, $C_{2-6}$ alkenyl, $C_{5-7}$ cycloalkylmethyl, substituted or unsubstituted phenyl or substituted or unsubstituted benzyl,

characterised in that a compound of formula IIb

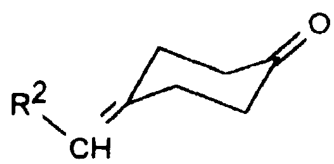
,

which is prepared according to claim 8, is hydrogenated in the presence of a Pd or Pt catalyst to form a 4-substituted cyclohexan-1-one of formula IIC

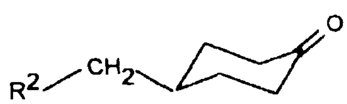

the compound of formula IIc which is obtained is reacted, either with dimethylamine in a Mannich reaction or with dimethylammonium methylene chloride, to form a compound of formula VIIc

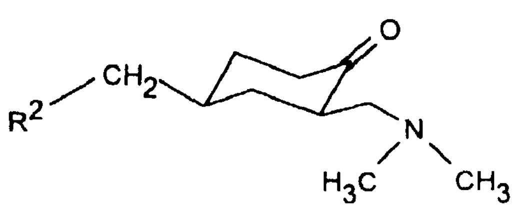

which is converted into a compound of formula I with an organometallic compound of formula VIII

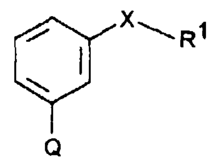

in which Q represents MgCl, MgBr, MgI or Li.

**12.** A method of preparing a 1-phenyl-2-dimethylaminomethyl-cyclohexan-1-ol compound of formula I

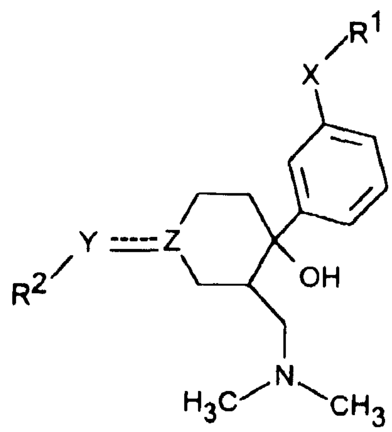

in which

X represents O or S, $R^1$ represents H,
the

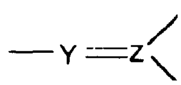

grouping represents

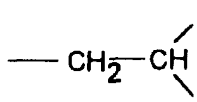 , 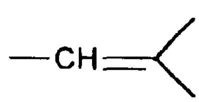 or 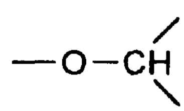

$R^2$ represents $C_{1-6}$ alkyl, $C_{2-6}$ alkenyl, $C_{5-7}$ cycloalkylmethyl, substituted or unsubstituted phenyl or substituted or unsubstituted benzyl,

characterised in that
either a compound of formula I, in which $R^1$ represents methyl, is reacted with diisobutylaluminium hydride, or a compound of formula I, in which $R^1$ is benzyl, is hydrogenated in the presence of a Pt or Pd catalyst,
or a compound of formula I, in which $R^1$ represents diarylalkylsilyl or trialkylsilyl, is hydrolysed or reacted with tetra-n-butylammonium fluoride.

**13.** A method of preparing a 1-phenyl-2-dimethylaminomethyl-cyclohexan-1-ol compound of formula I

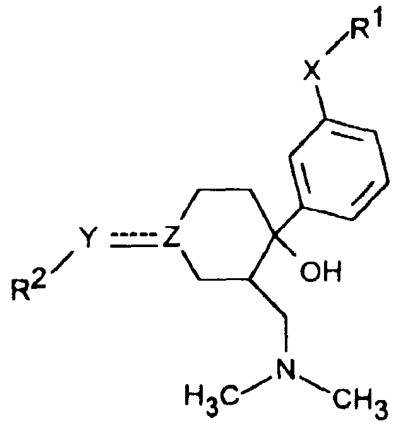

in which

X represents O or S,

$R^1$ represents H, $C_{1-6}$ alkyl, $C_{2-6}$ alkenyl, $C_{5-7}$ cycloalkyl, halogenated $C_{1-6}$ alkyl, benzyl, diarylalkylsilyl or triarylalkylsilyl,
the

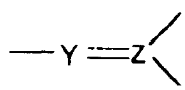

grouping represents

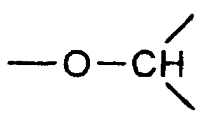 , 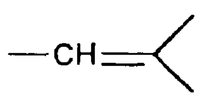 or 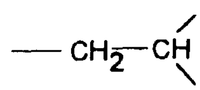

$R^2$ represents $C_{1-6}$ alkyl, $C_{2-6}$ alkenyl, $C_{5-7}$ cycloalkylmethyl, substituted or unsubstituted phenyl or substituted or unsubstituted benzyl,

characterised in that a ketone of formula XV

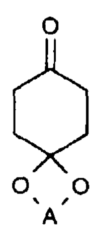 ,

in which A is a branched or unbranched $C_nH_{2n}$ radical and n represents an integer between 2 and 6, is reacted with dimethylammonium methylene chloride to form a β-dimethylaminoketone of formula XVI

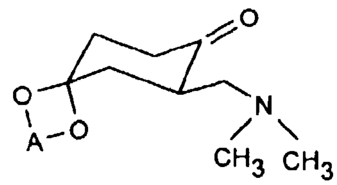

,

from which a compound of formula XVII

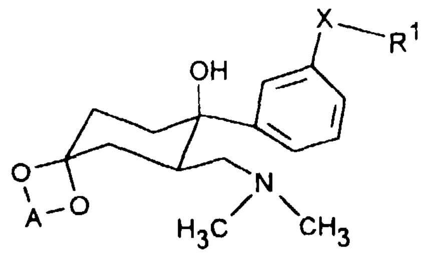

is subsequently prepared with an organometallic compound of formula VIII

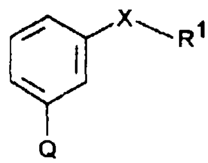

,

in which Q represents MgCl, MgBr, MgI or Li, which compound of formula XVII is subsequently deacetylated by means of proton catalysis to form a compound of formula XVIII

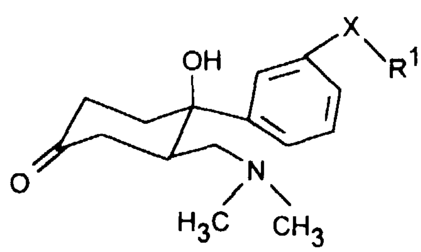

,

the compound of formula XVIII which is obtained is either reduced to form a 4-hydroxy derivative of formula XIX

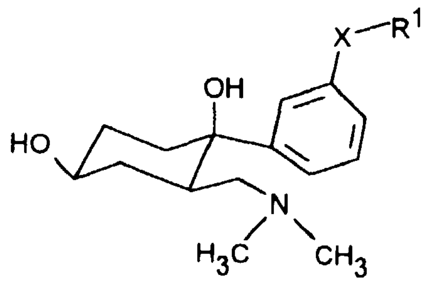

from which a compound of formula I is prepared, in which the

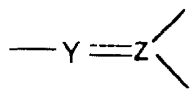

grouping is

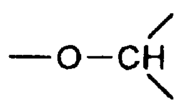
,

by subsequent alcoholate formation and reaction with a compound of formula III,

$R^2$--G,

in which G is Cl, Br, I or toluenesulphonyloxy,
or the compound of formula XVIII which is obtained is reacted with a compound of formula XX

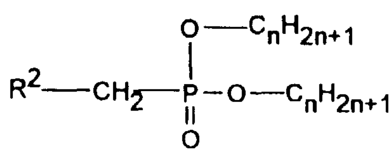

in which n represents an integer from 1 - 3, to form a compound of formula I, in which the

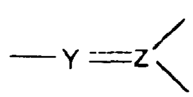

grouping is

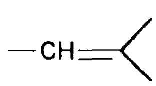
,

which is hydrogenated if desired to form a compound of formula I in which

the

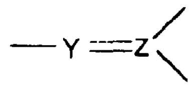

grouping is

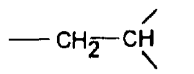

**14.** The use of a 1-phenyl-2-dimethylaminomethyl-cyclohexan-1-ol compound of formula I according to claim 1 for the production of a drug.

**15.** The use according to claim 12, characterised in that the drug is a pain-killing drug.

## Revendications

**1.** Composés de 1-phényl-2-diméthylaminométhyl-cyclohexane-1-ol de formule I

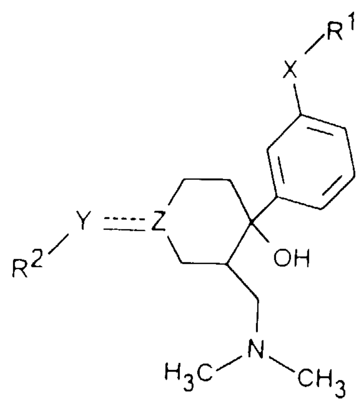

dans laquelle

X représente O ou S,
$R^1$ représente H, un groupe alkyle en $C_1$ à $C_6$, alcényle en $C_2$ à $C_6$, cycloalkyle en $C_5$ à $C_7$ ou alkyle en $C_1$ à $C_6$ halogéné,

le groupement

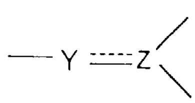

représente

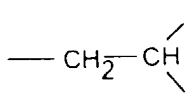 , 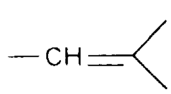 ou 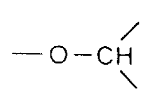

$R^2$ est un groupe alkyle en $C_1$ à $C_6$, alcényle en $C_2$ à $C_6$, (cycloalkyle en $C_5$ à $C_7$)-méthyle, phényle substitué ou non substitué ou benzyle substitué ou non substitué,

sous forme de leurs bases ou de leurs sels d'acides acceptables du point de vue physiologique.

**2.** Composés de 1-phényl-2-diméthylaminométhyl-cyclohexane-1-ol de formule I selon la revendication 1, caractérisés en ce que

$R^1$ représente H, un groupe alkyle en $C_1$ à $C_4$, 2'-méthyl-2'-propényle, cyclopentyle ou fluoréthyle, sous réserve que $R^1$ soit un groupe alkyle en $C_1$ à $C_4$, lorsque X représente S,
$R^2$ est un groupe alkyle en $C_1$ à $C_4$, alcényle en $C_2$ à $C_4$, cyclopentylméthyle, phényle, (alkoxy en $C_1$ à $C_4$) phényle, benzyle, (alkyle en $C_1$ à $C_4$)benzyle, phényle monohalogéné ou dihalogéné ou benzyle monohalogéné ou dihalogéné.

**3.** Composés de 1-phényl-2-diméthylaminométhyl-cyclohexane-1-ol de formule I suivant la revendication 1 ou 2, caractérisés en ce que :

$R^1$ représente H, un groupe méthyle, éthyle, isopropyle, 2'-méthyl-2'-propényle, cyclopentyle ou fluoréthyle, sous réserve que $R^1$ soit un groupe méthyle lorsque X représente S,
$R^2$ est un groupe méthyle, propyle, 2'-méthyl-propyle, allyle, 2'-méthyl-2'-propényle, cyclopentylméthyle, phényle, 3-méthoxyphényle, benzyle, 4-tertio-butyl-benzyle, 4-chlorobenzyle, 4-fluorobenzyle, ou 3,4-dichlorobenzyle.

**4.** Composés de 1-phényl-2-diméthylaminométhyl-cyclohexane-1-ol de formule I suivant l'une des revendications 1 à 3, caractérisés en ce que :

$R^1$ représente H, un groupe méthyle ou cyclopentyle, sous réserve que $R^1$ soit un groupe méthyle lorsque X représente S,

le groupement

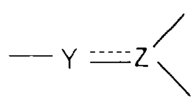

représente

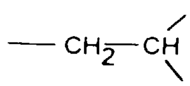

ou

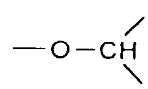

et

$R^2$ représente les groupes cyclopentylméthyle, benzyle et 4-chlorobenzyle.

**5.** Composés de 1-phényl-2-diméthylaminométhyl-cyclohexane-1-ol de formule I suivant la revendication 4, caractérisés en ce que X représente O, $R^1$ représente H ou un groupe méthyle,
le groupement

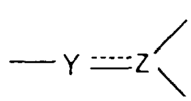

représente

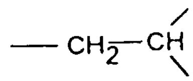

ou

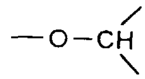

et

$R^2$ est un groupe benzyle.

**6.** Composés de 1-phényl-2-diméthylaminométhyl-cyclohexane-1-ol suivant l'une des revendications 1 à 5, caractérisés en ce qu'ils ont la configuration de la formule Ia :

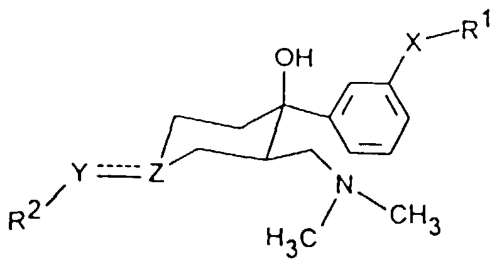

**7.** Médicament contenant comme substance active au moins un composé selon les revendications 1 à 6 et le cas échéant d'autres substances actives.

**8.** Médicament selon la revendication 7, ayant une action analgésique.

**9.** Procédé de production d'un composé de 1-phényl-2-diméthylaminométhyl-cyclohexane-1-ol de formule I

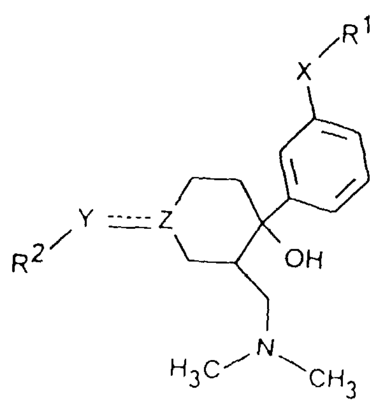

dans laquelle

X représente O ou S,
$R^1$ est un groupe alkyle en $C_1$ à $C_6$, alcényle en $C_2$ à $C_6$, cycloalkyle en $C_5$, à $C_7$, alkyle en $C_1$ à $C_6$ halogéné, benzyle, diarylalkylsilyle ou trialkylsilyle,

le groupement

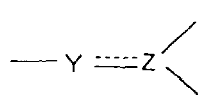

représente

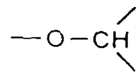

$R^2$ est un groupe alkyle en $C_1$ à $C_6$, alcényle en $C_2$ à $C_6$, (cycloalkyle en $C_5$ à $C_7$)méthyle, phényle substitué ou non substitué ou benzyle substitué ou non substitué,

caractérisé en ce qu'on prépare une cyclohexane-1-one substituée en position 4, de formule IIa :

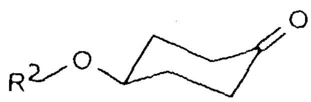

en alkylant un composé de formule IV :

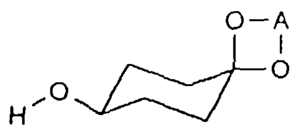

dans laquelle A est un reste $C_nH_{2n}$ ramifié ou non ramifié, et n est un nombre entier de valeur comprise entre 2 et 6, avec un composé de formule III :

$$R^2\text{-G}$$

dans laquelle G représente Cl, Br, I ou un groupe toluène-sulfonyloxy, puis en désacétalisant le produit par catalyse protonique,
ou bien en alkylant un composé de formule V :

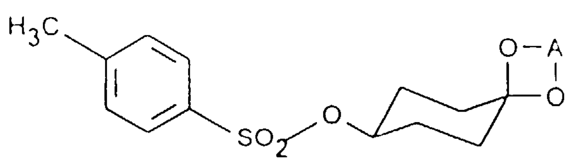

dans laquelle A est un reste $C_nH_{2n}$ ramifié ou non ramifié, et n est un nombre entier entre 2 et 6, avec un alcoolate que l'on prépare à partir d'un alcool de formule VI

$$R^2\text{-OH}$$

puis en désacétalisant le produit par catalyse protonique en un composé de formule IIa,
on fait réagir le composé obtenu de formule IIa avec la diméthylamine dans une réaction de Mannich, ou avec le chlorure de diméthylammonium-méthylène, pour former un composé de formule VIIa :

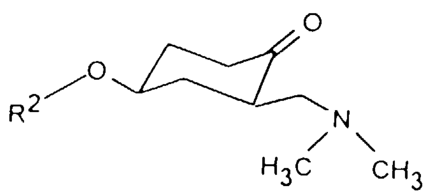

que l'on transforme avec un composé organométallique de formule VIII :

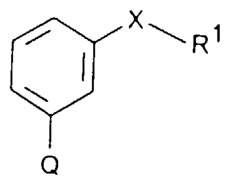

dans laquelle Q représente MgCl, MgBr, MgI ou Li, en un composé de formule I.

**10.** Procédé de production d'un composé de 1-phényl-2-diméthyl-aminométhyl-cyc1ohexane-1-ol de formule I

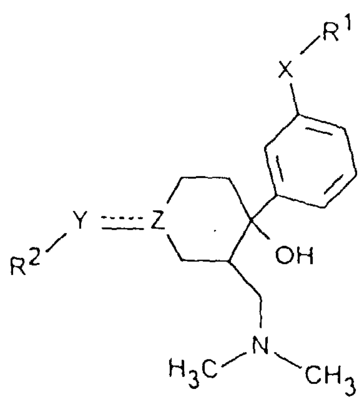

dans laquelle

X représente O ou S,
$R^1$ est un groupe alkyle en $C_1$ à $C_6$, alcényle en $C_2$ à $C_6$, cycloalkyle en $C_5$ à $C_7$, alkyle en $C_1$ à $C_6$ halogéné, benzyle, diarylalkylsilyle ou trialkylsilyle,

le groupement

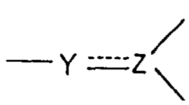

représente

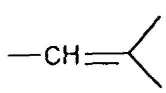

$R^2$ est un groupe alkyle en $C_1$ à $C_6$, alcényle en $C_2$ à $C_6$, (cycloalkyle en $C_5$ à $C_7$)méthyle, phényle substitué ou

non substitué, ou benzyle substitué ou non substitué,

qui est caractérisé en ce qu'on prépare une cyclohexane-1-one substituée en position 4 de formule IIb :

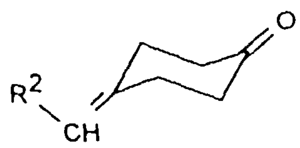

en faisant réagir un composé de Grignard de formule IX

$$R^2\text{-}CH_2\text{-Mg-Hal}$$

dans laquelle Hal représente Cl, Br ou I, avec une cétone de formule X :

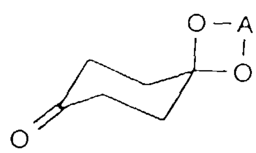

dans laquelle A est un reste $C_nH_{2n}$, ramifié ou non ramifié et n est un nombre entier de valeur comprise entre 2 et 6, pour obtenir un composé de formule XI :

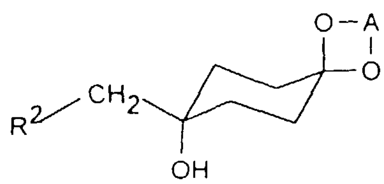

à partir duquel on prépare ensuite par désacétalisation catalysée par des protons, un composé cétonique de formule XII :

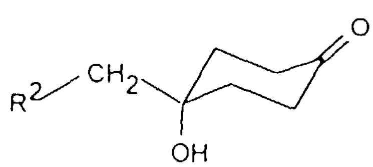

et on obtient par déshydratation subséquente le composé de formule IIb
ou bien en faisant réagir une cétone de formule X avec un phosphorane de formule XIII :

$$R_3P{=}CH\text{-}R^2$$

dans laquelle R est un groupe aryle, selon une réaction de Wittig, pour obtenir un composé de formule XIV :

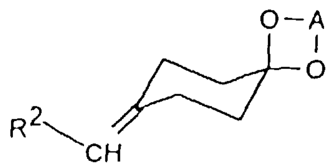

dans laquelle A est un reste $C_nH_{2n}$ ramifié ou non ramifié et n est un nombre entier compris entre 2 et 6, qu'on transforme ensuite par catalyse protonique en une cétone de formule IIb,
on fait ensuite réagir le composé obtenu de formule IIb avec la diméthylamine dans une réaction de Mannich, ou avec le chlorure de diméthyl-ammonium-méthylène, pour obtenir un composé de formule VIIb :

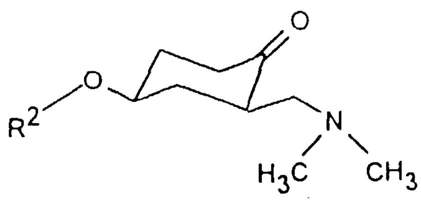

que l'on transforme avec un composé organométallique de formule VIII

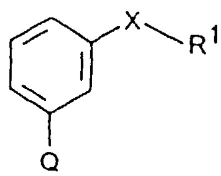

dans laquelle Q représente MgCl, MgBr, MgI ou Li, en un composé de formule I.

**11.** Procédé de production d'un composé de 1-phényl-2-diméthylaminométhyl-cyclohexane-1-ol de formule I

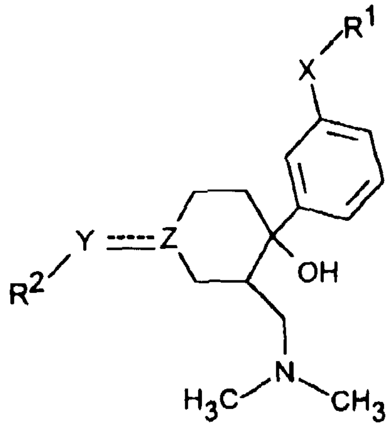

dans laquelle

X représente O ou S,
$R^1$ est un groupe alkyle en $C_1$ à $C_6$, alcényle en $C_2$ à $C_6$, cycloalkyle en $C_5$ à $C_7$ alkyle en $C_1$ à $C_6$, halogéné, benzyle, diarylalkylsilyle ou trialkylsilyle,

le groupement

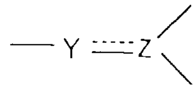

représente

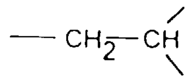

$R^2$ est un groupe alkyle en $C_1$ à $C_6$, alcényle en $C_2$ à $C_6$, (cycloalkyle en $C_5$ à $C_7$)-méthyle, phényle substitué ou non substitué, ou benzyle substitué ou non substitué,

caractérisé en ce qu'on hydrogène un composé préparé selon la revendication 10, de formule IIb :

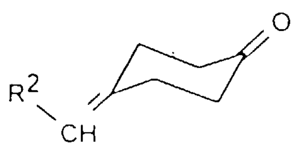

en présence d'un catalyseur au palladium ou au platine, pour obtenir une cycloexane-1-one substituée en position 4 de formule IIc :

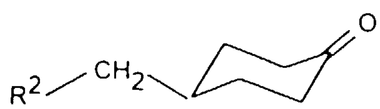

on fait réagir le composé de formule IIc obtenu avec la diméthylamine dans une réaction de Mannich ou avec le chlorure de diméthylammoniumméthylène, pour obtenir un composé de formule VIIc :

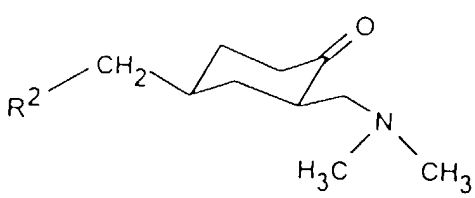

que l'on transforme avec un composé organométallique de formule VIII

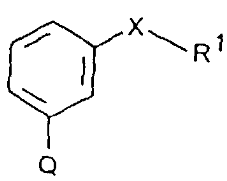

dans laquelle Q représente MgCl, MgBr, MgI ou Li, en un composé de formule I.

**12.** Procédé de production d'un composé de 1-phényl-2-diméthylaminométhyl-cyclohexane-1-ol de formule I :

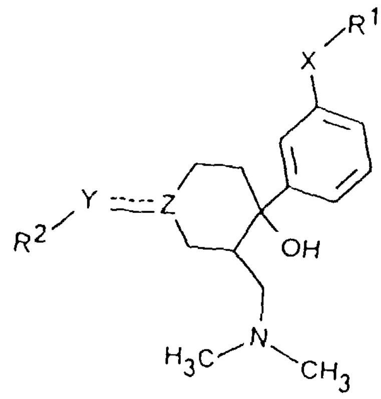

dans laquelle X représente O ou S, $R^1$ représente H,
le groupement

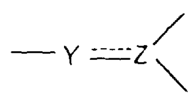

représente

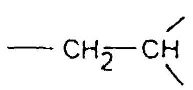 , 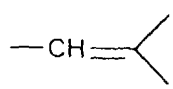 ou 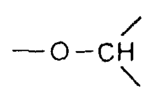

$R^2$ est un groupe alkyle en $C_1$ à $C_6$, alcényle en $C_2$ à $C_6$, (cycloalkyle en $C_5$ à $C_7$)méthyle, phényle substitué ou non substitué, ou benzyle substitué ou non substitué,

caractérisé en ce qu'on fait réagir un composé de formule I, dans laquelle $R^1$ est un groupe méthyle, avec l'hydrure de diisobutylaluminium, ou bien on hydrogène un composé de formule I, dans laquelle $R^1$ est un groupe benzyle, en présence d'un catalyseur au platine ou au palladium,
ou bien on hydrolyse un composé de formule I, dans laquelle $R^1$ est un groupe diarylalkylsilyle ou trialkylsilyle, ou on le fait réagir avec le fluorure du tétra-n-butylammonium.

**13.** Procédé de production d'un composé de 1-phényl-2-diméthyl-aminométhyl-cyclohexane-1-ol de formule I

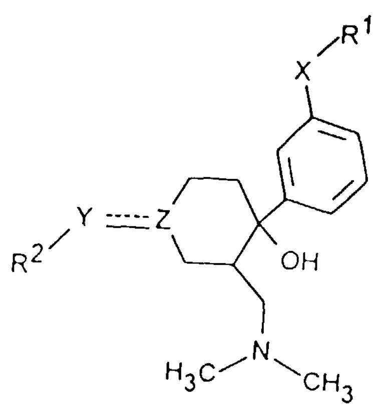

dans laquelle

X représente O ou S,

$R^1$ représente H, un groupe alkyle en $C_1$ à $C_6$, alcényle en $C_2$ à $C_6$, cycloalkyle en $C_5$ à $C_7$, alkyle en $C_1$ à $C_6$ halogéné, benzyle, diarylalkylsilyle, ou trialkylsilyle,

le groupement

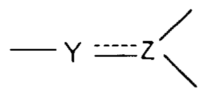

représente

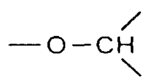 , 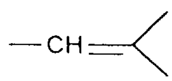 ou 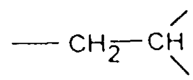

$R^2$ est un groupe alkyle en $C_1$ à $C_6$, alcényle en $C_2$ à $C_6$, (cycloalkyle en $C_5$ à $C_7$)-méthyle, phényle substitué ou non substitué, ou benzyle substitué ou non substitué,

caractérisé en ce qu'on fait réagir une cétone de formule XV :

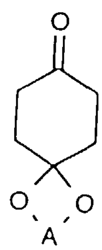

dans laquelle A est un reste $C_nH_{2n}$ ramifié ou non ramifié, et n est un nombre entier compris entre 2 et 6, avec le chlorure de diméthylammonium-méthylène pour obtenir une β-diméthylaminocétone de formule XVI :

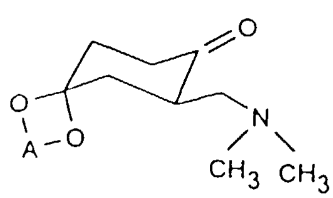

à partir de laquelle on prépare ensuite avec un composé organométallique de formule VIII

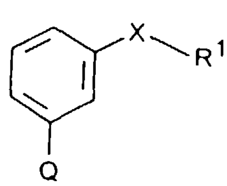

dans laquelle Q représente MgCl, MgBr, MgI, ou Li, un composé de formule XVII :

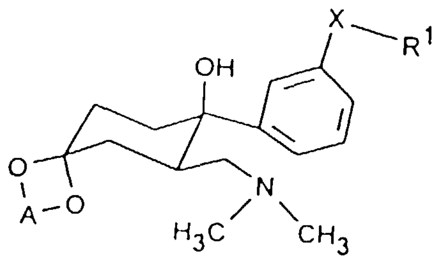

que l'on désacétalise ensuite par catalyse protonique en un composé de formule XVIII :

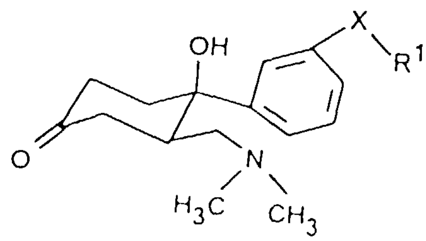

on réduit le composé obtenu de formule XVIII en un dérivé 4-hydroxy de formule XIX :

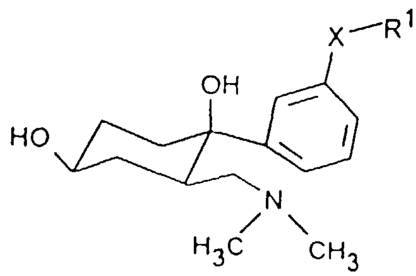

à partir duquel on prépare par formation subséquente de l'alcoolate et réaction avec un composé de formule III,

$$R^2\text{-}G$$

dans laquelle G représente Cl, Br, I ou un groupe toluènosulfonyloxy, un composé de formule I, dans laquelle le groupement

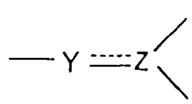

représente

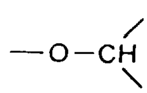

ou bien on fait réagir le composé obtenu de formule XVIII avec un composé de formule XX :

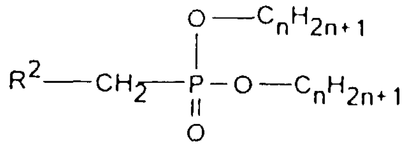

dans laquelle n est un nombre entier de 1 à 3, pour obtenir un composé de formule I, dans laquelle le groupement

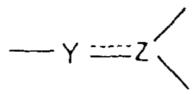

représente

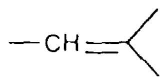

que l'on hydrogène le cas échéant en un composé de formule I, dans laquelle le groupement

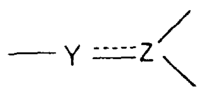

représente

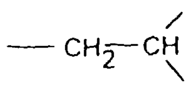

**14.** Utilisation d'un composé de 1-phényl-2-diméthylaminométhyl-cyclohexane-1-ol de formule I suivant la revendication 1 pour la préparation d'un médicament.

**15.** Utilisation suivant la revendication 14, caractérisée en ce que le médicament est un analgésique.